# EUROPEAN PATENT SPECIFICATION

(11) **EP 2 148 721 B1**
(45) Date of publication and mention of the grant of the patent: **30.07.2014**
(21) Application number: 08767759.7
(22) Date of filing: 16.05.2008
(51) Int. Cl.: A61N 1/30, A61N 1/32

(54) **DEVICE AND METHOD FOR SINGLE-NEEDLE IN VIVO ELECTROPORATION**
VORRICHTUNG UND VERFAHREN ZUR IN-VIVO-EINZELNADELELEKTROPORATION
DISPOSITIF ET PROCÉDÉ POUR ÉLECTROPORATION IN VIVO À AIGUILLE UNIQUE

(30) Priority: 18.05.2007 US 804703; 20.08.2007 US 894653
(43) Date of publication of application: 03.02.2010
(73) Proprietor: Genetronics, Inc., San Diego, CA 92121-1318 (US)
(72) Inventor: KJEKEN, Rune, San Diego, CA 92121-1318 (US); MATHIESEN, Iacob, San Diego, CA 92121-1318 (US); TJELLE, Torunn Elisabeth, San Diego, CA 92121-1318 (US); MCHUGH, George, San Diego, CA 92121-1318 (US)
(74) Representative: Bassil, Nicholas Charles
(86) International application number: PCT/US2008/006311
(87) International publication number: WO 2008/143988

(56) References cited:
- US-A- 5 389 069
- US-A- 6 135 990
- US-A1- 2005 070 841
- US-A1- 2005 070 841
- US-A1- 2005 123 565
- US-A1- 2006 089 674
- US-A1- 2006 293 725
- US-A1- 2007 083 239
- US-B1- 6 591 133
- US-B1- 6 603 998
- US-B1- 6 778 853
- US-B1- 6 778 853

## Description

This invention relates to electroporation of cells *in vivo,* particularly cells of a patient's tissues. More specifically, this invention relates to novel devices and methods for delivering molecules to cells situated at, near and/or adjacent to a predetermined insertion track site of an elongate single-needle electrode. Still more specifically, the invention concerns the electroporated delivery of substances into cells along and in the vicinity of the needle track made by insertion of the electrode from the surface of a tissue and into the tissue to a depth of from about 3 millimeters to about 3 cm, which tissues can comprise any tissues, including without limitation skin, epidermis, dermis, hypodermis, connective tissue, striated and smooth musculature throughout tissue layers of the body, mucosa, and organs.

### BACKGROUND OF THE INVENTION

The following description includes information that may be useful in understanding the present invention. It is not an admission that any such information is prior art, or relevant, to the presently claimed inventions, or that any publication specifically or implicitly referenced is prior art.

Electroporation has been applied to delivering molecules to subsurface tissues using various multiple-electrode designs such as arrays of two or more electrodes that typically are designed as needle electrodes for insertion into said tissue. Generally, such arrays define a treatment zone lying between the needle electrodes of the array. Such treatment zones therefore comprise a three dimensional volume of tissue wherein cells within the treatment zone are exposed to an electric field of an intensity sufficient to cause temporary or reversible poration, or even sometimes irreversible poration, of the cell membranes to those cells lying within and or near the three dimensional volume.

Current practices for electroporating cells in tissue include use of significant voltages in order to impart through the three dimensional treatment zone a relatively uniform electric field. By "relatively uniform" is meant that electric lines of force coincident with application of an electric pulse sufficient to cause poration is imparted across the cells somewhat evenly throughout the three dimensional treatment zone volume. Ultimately, a large number of electrode needles combined with large injection volumes and high electrical fields have been necessary to ensure a sufficient overlap between an injected drug and the tissue volume experiencing the electrical field since typically, the injection bolus that is delivered to the tissues quickly spreads from the injection site. Use of high electric fields and large electrode arrays has several drawbacks. For example, use of many needles and high electric field (voltages) causes more pain while high injection volume makes dosing difficult to control as it causes waste of the drug (most of the drug is not getting into the cells as it will be outside the treatment zone). Also, use of such multiple needle devices is cumbersome and a cause for apprehension from the standpoint of the patient.

US 6778853 describes a device for manipulating a molecule *in vivo* relative to a target tissue including at least one elongated member having at least two discrete and separately activatable electrodes separated by an insulating material interposed therebetween.

Besides the invasive aspect of a device with multiple needles, typical electroporation techniques, as stated above, result in variability in electroporation of cells within a treatment zone. This is a drawback to medical use of electroporation in that dispersion of treatment molecules of the injected bolus into surrounding tissue results in loss of control as to the amount of such treatment molecule that is ultimately transfected into cells within the treatment zone by the electroporation event. Thus, a need exists in the electroporation arts for a device and method to narrow or refine control over "dosing" of treatment molecules into specific and well-defined delivery sites within a patient's tissue. Likewise, there is still a need in the art for methodologies and devices that can electroporate with less invasiveness and impart less pain from the electric field pulse employed in the delivery of therapeutic substances to various tissues including skin, muscle, mucosa and organs.

### SUMMARY OF THE INVENTION

The subject matter for which protection is sought is as defined in the claims.

In a first aspect, this invention provides for electroporation of cells *in situ,* particularly cells that are located subcutaneously, intradermally, subdermally, and/or intramuscularly (particularly striated skeletal muscle, striated muscle under the skin, cardiac muscle, and smooth muscle). In this aspect, the invention provides for the electroporation of cells near and/or adjacent to the track made by insertion of an elongate single-needle electrode into tissue. In preferred embodiments, cells that become electroporated using the invention device are those situated within a radius from the needle track anywhere from between 0.0mm (millimeter) to about 5mm or even 6 mm so as to comprise a generally cylindrical treatment zone imparted by the novel electrode design and pulsing of the electric field imparted into the tissue by the single-needle electrode when it is energized by a power supply.

The invention provides for any suitable structural arrangement of the anode(s) and cathode(s) on a single-needle electrode according to the invention such that there are at least two electrically opposite electrically conductive leads (i.e., at least one anode and at least one cathode) situated in association with a single elongate shaft, which shaft itself is constructed to be electrically inert. In one embodiment said single-needle electrode can comprise anode(s) and cathode(s) and an electrically inert material, such as a medically acceptable plastic or polycarbonate, filling the space between the anode(s) and cathode(s) or an electrically inert coating on an elongate rigid material that itself is electrically conductive, such as a metallic hypodermic needle. In a preferred embodiment the shaft upon which the anode(s) and cathode(s) are situated can have cross sectional dimensions of between about 0.05 mm to about 1.5 mm.

The single-needle electrode comprises a combination of opposing elongate tissue piercing anode and a cathode spaced and electrically insulated from one another but with no inert material there between. In such an embodiment the spaced anode and cathode run substantially parallel to one another each having a distal and a proximal end and positioned relative to one another such that the distal ends lie opposite one another and the proximate ends are held one to another by an electrically inert substrate. In either embodiment, the anode(s) and cathode(s) of the tissue penetrating single-needle electrode can be spaced between about 0.05 mm and about 1.5 mm.

In a related embodiment, the electrodes themselves can have a length exposed along the elongate shaft anywhere from the whole single-needle electrode length to only a portion of the single-needle shaft, such as near the shaft penetration tip.

In another embodiment, the electrodes can have cross sectional dimensions of between about 0.005 and 0.80 mm or thereabout.

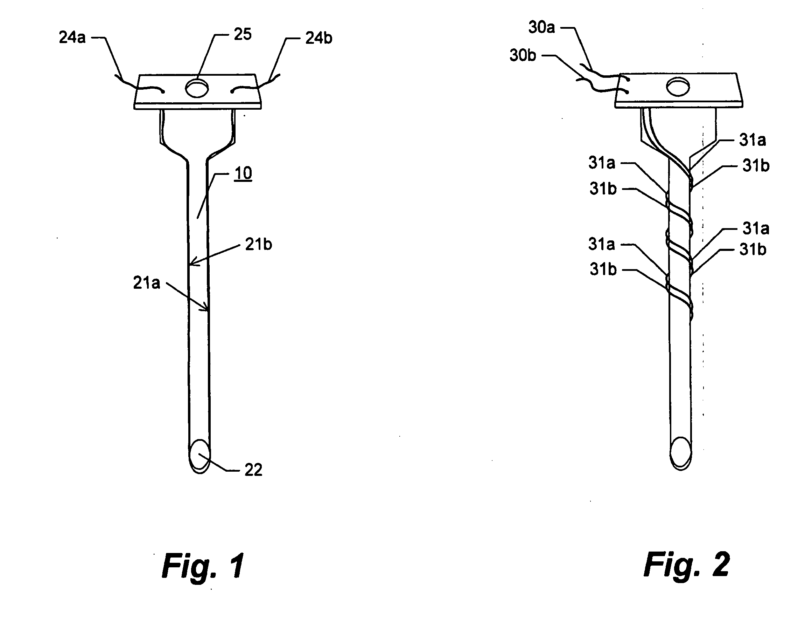

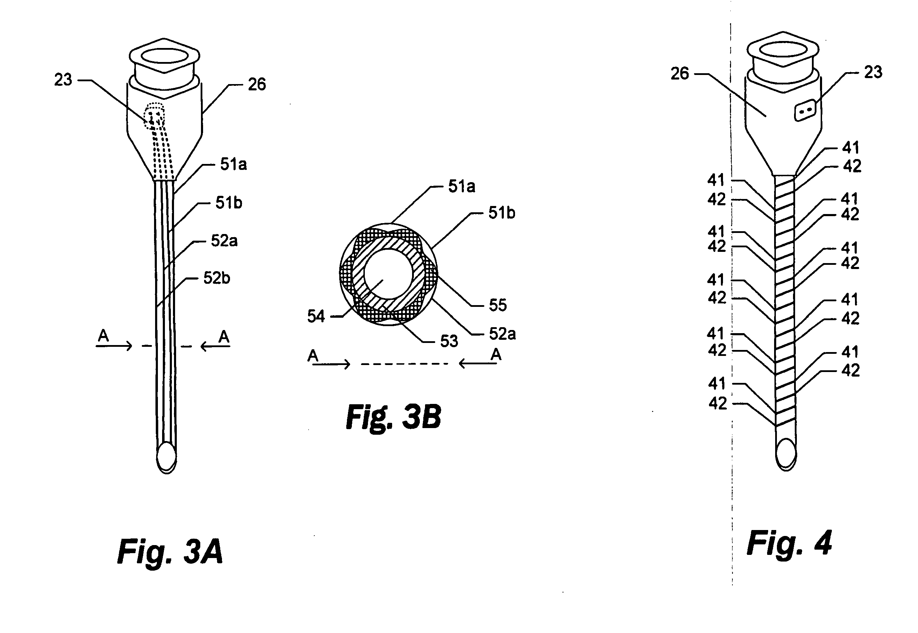

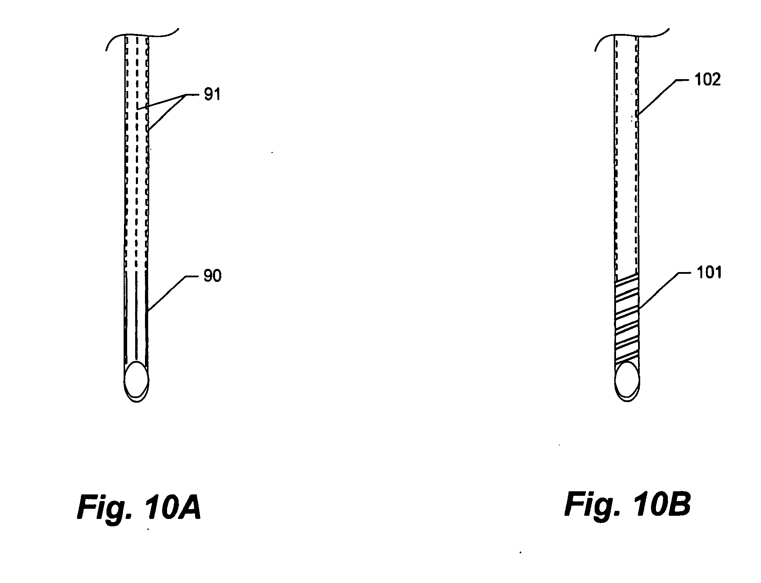

In yet another structural arrangement embodiment, the single-needle electrode can comprise a hypodermic needle comprising at least two elongate electrodes spaced along at least a portion of the length of the hypodermic needle exterior. For example, the hypodermic needle can include at least two electrodes (i.e., an anode and a cathode) running along a portion of the length of the needle. See Figure **10A**. In other examples, multiple electrodes can be formed on the exterior of a hypodermic injection needle such as disclosed in Figures **3A** comprising multiple straight and parallel electrodes, or as depicted in Figures **2** and **4** comprising multiple electrodes spiraled around the injection needle. In working embodiments, each anode and cathode is connected to a source of electric energy for generating an electric field around the single-needle shaft.

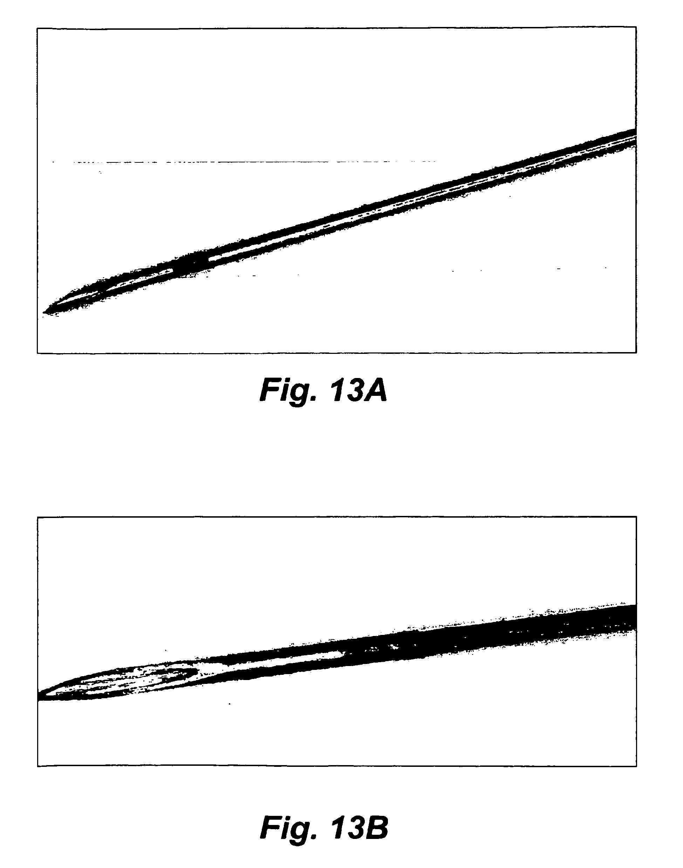

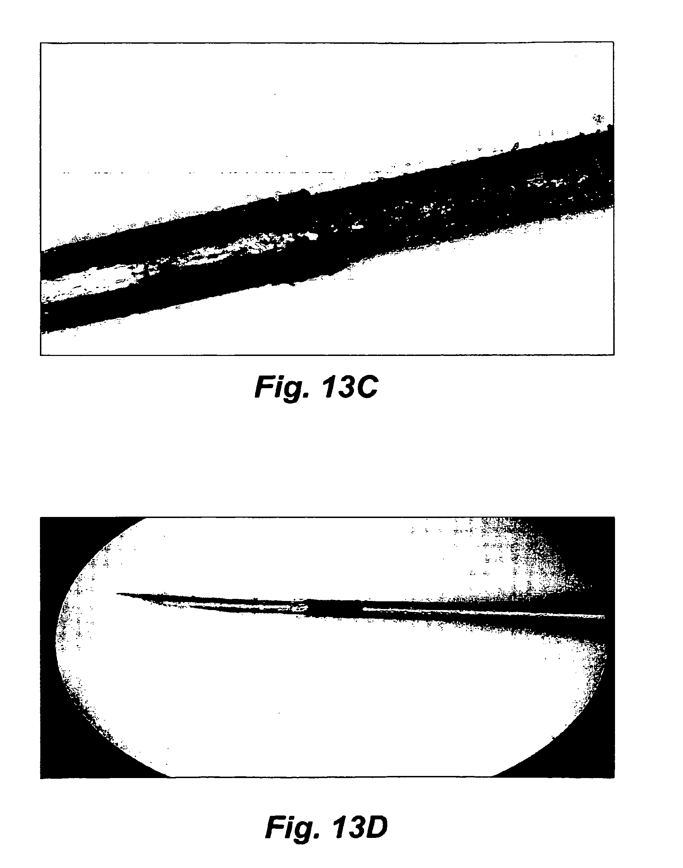

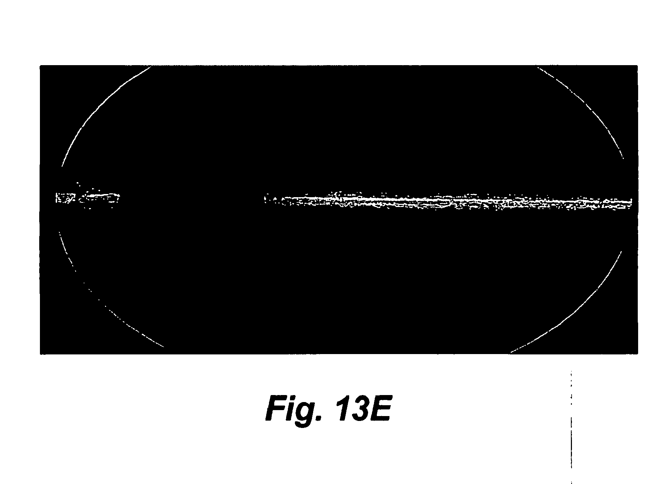

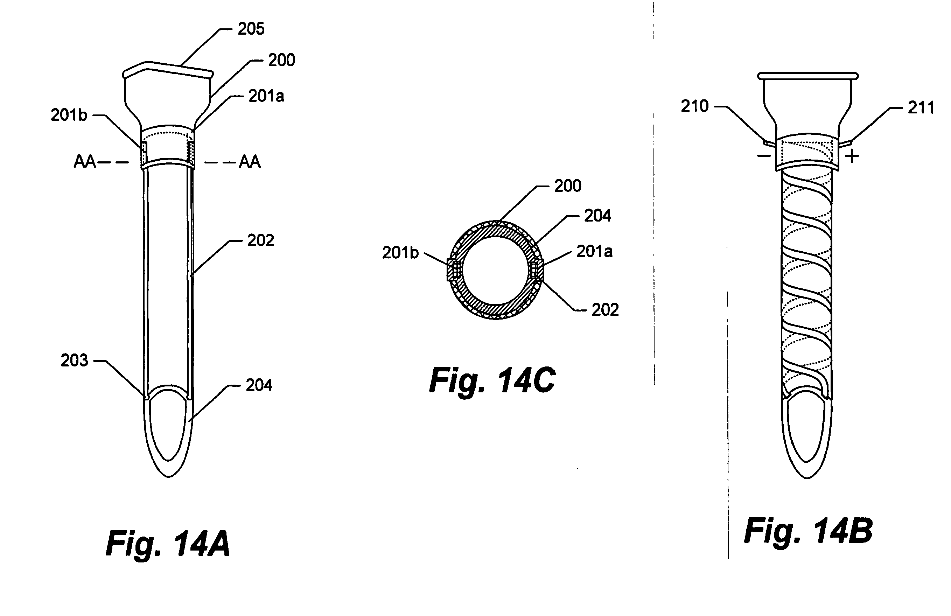

In still further embodiments, the single-needle electrodes can be manufactured using any number of well understood methods, including etching and layering per Micro ElectroMechanical Systems (MEMS) technologies. In such manufacturing methods, micromachining processes are used to add or strip away layers of substances important to the proper annealing, insulation, and conduct of electric pulses and circuitry. Figures **13A****, B, C, D** and **E** are photographs of the embodiment wherein the electrodes are etched on to the delivery single-needle shaft. Specifically, gold electrode layering has been coated above a layer of and inert substance, such as parylene, which itself has been layered over the hypodermic needle shaft. Additional methods for manufacturing the elongate electrodes include extrusion technologies wherein the anode and cathode leads are formed into and/or along the shaft of an electrically inert composition having insulating qualities, such a plastic, a polyester derivative, or polyvinylchloride (PVC), or insulative carbon fiber. As shown in Figure **14** **A** and **B,** an elongate hollow needle can be extruded with anode and cathode components, such as for example, wire either along opposite sides of a hollow shaft or in a spiral fashion as shown in Figure **14** **B.**

Further still, the needle shaft can also comprise sections with no exposed electrodes as disclosed in Figures **10A** and **B****.** For example, one end of the needle shaft connects to a hub forming a connector for connecting to a source of fluid, such as for example, a syringe. Insulation near or along such section of the shaft may provide for additional lessening of electric stimulus sensation noticeable by the patient. In yet a further embodiment with respect to any such single-needle electrode configuration described herein, each of the anodes and cathodes are individually energizable so that any combination of the anodes and cathodes may be energized in pairs (i.e., a cathode and an anode) simultaneously together, or in any given sequence, and further using any type of pulse including without limitation monopolar, bipolar, exponential decaying, or pulse train combinations of any of the former.

In a related aspect, the invention provides for use of relatively low voltage and/or low current, which in turn not only provides sufficient electrical energy for causing reversible poration of cells in the treatment zone, but also allows for a low pain level experienced by subjects during application of electric pulses into the tissue surrounding the insertion site, said application using nominal electric field strengths of generally between about 1 and about 100 V, typically between 5 and 75V, an more preferably between 10 and 50V. In another related aspect, electric current employed in the invention device and methods uses generally between 1-1000 mAmps (milliamps), typically between about 10-500 mAmps, and more preferably between about 20 and about250 mAmps. In a related embodiment, the amperage chosen depends on the total surface area of the electrodes. For example, the single-needle electrode may employ a range between 10 to 40, or 25 to 100, or 50 to 150, or 125 to 200, or 175 to 250, or 225 to 300, or 250 to 300, 300 to 400, 400-600, or 600 to 1000 mAmps depending upon the total surface area of each of the anode(s) and cathode(s). The smaller the surface area, the lower the amperage necessary to achieve an electroporating electric filed in the *in situ* tissue. Pulses sequences of any desired number of pulses, pulse duration, and delays between pulses can be employed, with pulse durations of from between 0.1 and 1000 millisec. or thereabout being preferred.

The present disclosure provides for delivery of treatment molecules in solution (i.e., a therapeutic liquid composition) at various concentrations (e.g., for example, between about 0.05 µg-3 mg/ml) and preferably at low bolus volumes (e.g., for example, generally 1µl to 1ml). In a related embodiment, using a structural embodiment inclusive of a delivery tube associated with the single-needle electrode shaft, the volume of therapeutic liquid composition containing the treatment molecules (also referred to herein as "active ingredients") immediately following injection into the tissue (such as a controlled injection wherein the injectate is delivered during insertion of the needle) surprisingly remains to a substantial degree in the vicinity of the needle shaft insertion track. Also contemplated with respect to the injection of material to be delivered in the vicinity of the needle track is the use of fenestrated needles wherein there are one or more delivery ports spaced along the length of the single-needle electrode delivery tube shaft (not shown but understandable to those of ordinary skill in the art). Treatment molecules (i.e., active ingredients) are contemplated to include any useful therapeutic drug or compound, e.g., small molecules, organic compounds, as well as proteins and peptides, and also nucleic acids, including those that code for the expression of a peptide, polypeptides, protein, or biologically active nucleic acid species (e.g., and antisense RNA, interfering RNA, ribozymes, other catalytic RNA species, etc.) having either a biologic activity or that will induce an immune response in the host once such polypeptide is expressed in the electroporated cell. The polypeptides once expressed in the cell are available for interacting with cellular metabolic machinery and immune system pathways.

In yet another embodiment, electrical energy used to pulse the tissue provides for a unique electric field that is unlike prior applied fields used for electroporation of similar tissues. Specifically, prior art electric fields intentionally and inherently impart what has been recognized in the electroporative arts as a "uniform" electric field, meaning that the applied electrical energy is of sufficient strength to impart a nominal field strength and a relatively even voltage drop across the treatment zone created by widely separating the electrodes a given distance apart from one another and placing the target treatment zone optimally central between said spaced electrodes. Such electrode array designs when pulsed in tissue tend to electroporate cells primarily within the zone surrounded by or between the electrodes generally in the vicinity of the electric lines of force and to a smaller degree a zone of cells situated just adjacent and surrounding the three dimensional treatment zone.

In contrast, the current invention uses electric fields that comprise a generally cylindrical or columnar "non-uniform" field that is created about the length of the single-needle shaft thereby creating a treatment zone of cells lying within an area close enough to the centrally placed anode(s) and cathode(s) to be subjected to an electroporation field "outside" the immediate location of the electrodes, of sufficient strength to porate said cells. Such a treatment zone is completely external to and surrounding the central single-needle shaft and the non-uniform field dissipates relative to the distance outward from the single-needle shaft. Generally, it is understood that the electroporative electrical energy dissipates as the distance from the single-needle electrode increases, such as, for example, dissipation at an exponential rate. However, such dissipation rate, if applicable, does not negatively affect the functioning of the invention device or the intended outcome of delivering substances into cells in a defined zone. Thus, since electrical energy necessary to cause cell poration dissipates with the distance from the electrical field source, the area around the needle tract that is susceptible to electroporation is inherently confined to a central core correlating to the length of the needle track and laterally to a given radius forming therefore a generally cylindrical treatment zone of variable radii depending upon the pulse energy imparted to the anode(s) and cathode(s). In a further related embodiment, the more energy used to pulse, the greater the potential to damage cells directly in contact with the electrodes. It is yet a further intention of the invention to employ the ability to cause such damage for the purpose of further stimulating the immune system. Thus, treatment regimens can be used that intentionally impart a greater, rather than a lesser, energy so as to provide a stimulus for immune response activity around the treatment site.

In other embodiments, the device can be used to deliver drugs such as natural polypeptides having a biologic activity and genes encoding such polypeptides that can be expressed *in situ* in cells within the treatment zone for treating disorders or for modulating an immune response in the host and/or for treating a variety of diseases including but not limited to diseases caused by pathogenic organisms and viruses and cancers.

Other features and advantages of the invention will be apparent from the following drawings, detailed description, and appended claims.

### BRIEF DESCRIPTION OF THE DRAWINGS

This specification contains at least one figure executed in color. Copies hereof with color drawing(s) will be provided upon request and payment of the necessary fee.
Figure **1** is a drawing depicting a hypodermic needle with elongate cathode and anode integrated therein. The needle features a port for dispensing a liquid formulation from a lumen running there through, and a port for connecting to a fluid-carrying reservoir.
Figure **2** depicts an alternate embodiment of the invention device wherein the anodes and cathodes are parallel to one another through a plane formed in a spiral around the needle.
Figure **3A** is another alternate embodiment wherein a delivery needle comprises a multiplicity of anode and cathode electrodes running straight and parallel along the length of the delivery needle. As also depicted, this figure includes an example of a connector for connecting the electrodes to a source of electrical energy. Figure **3B** depicts a view of the cross section of one example of an invention electrode along line A-A. As shown, in one configuration, the electrodes can be layered by any number of techniques known to those of skill in the fabrication arts on the outer sections of a delivery tube and lumen. In the drawing is depicted an inner needle **53** with lumen **54** surrounded by an insulating material **55** on which is layered the electrodes.
Figure **4** is another example of an embodiment comprising anodes and cathodes spiraled around the delivery needle. The anodes and cathodes so spiraled can comprise a multiplicity of anode and cathode pairs, but typically comprise one or two pairs, each pair comprising an anode and a cathode.

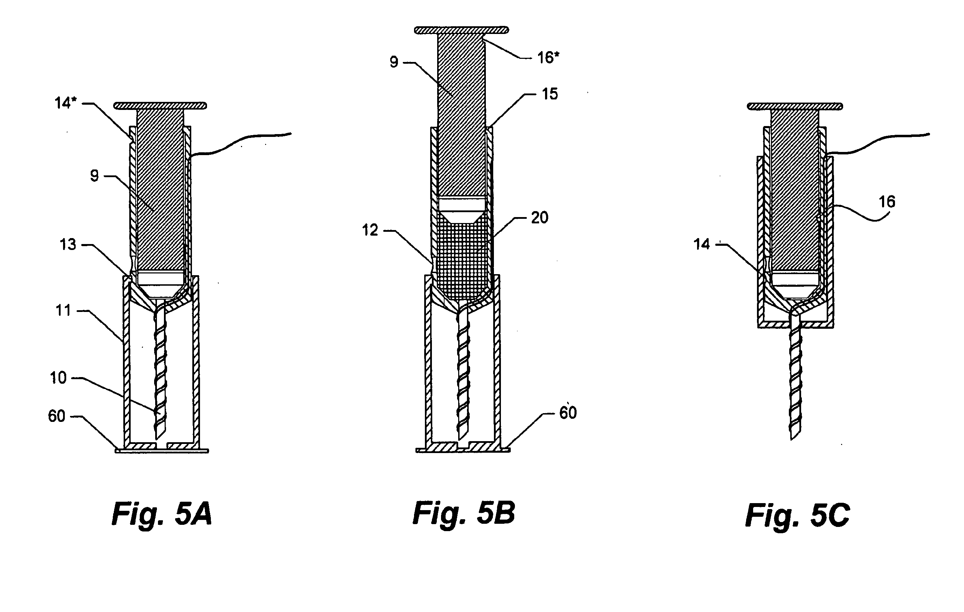

Figures **5A-C** depict one embodiment of the invention wherein the invention electrode is shown comprising further embodiments including a reservoir, typically a syringe styled reservoir, and a sharps cover which is capable of retracting as the needle is inserted into a patient tissue. The drawing also shows other features that can be embodied within the invention device such as a resilient membrane which can be pierced such as by a needle to fill the reservoir and mechanisms for allowing the sharps cover and the syringe plunger to be held in place either in an extended or retracted position. Moreover, the retractable sharps cover also act as a needle guide and can be fitted with stops to act as a depth guide. Although not shown, the single-needle electrode can be fitted to a syringe and attached to an automatic needle delivery/simultaneous fluid delivery electroporation device such as that depicted in US Patent Application US 2004/0059285 and WO 2004/004825. In such embodiment, the device would only have one single-needle electrode and one syringe.

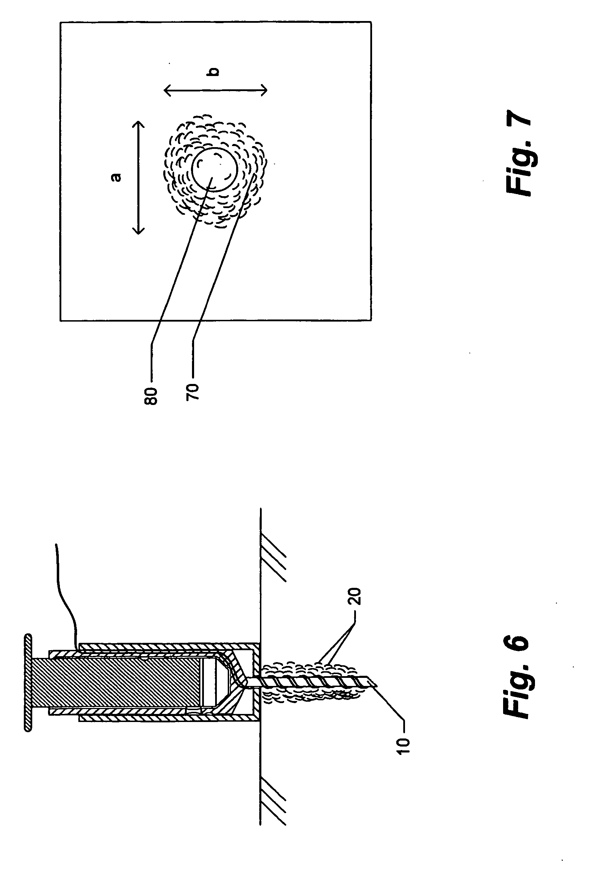

Figure **6** shows a depiction of the invention device in use wherein during insertion or after the electrode/delivery needle is inserted into the tissue, the fluid material administered, the anodes and cathodes are energized so as to impart an electric field outward from the needle track and into the tissue. The electric field dissipates outward into the tissue from the site of the inserted needle.
Figure 7 shows a top view of a hypothetical tissue and a depiction of typical electric field that the invention device would generate in the tissue surrounding the needle track and having lateral dimensions (a) and (b).

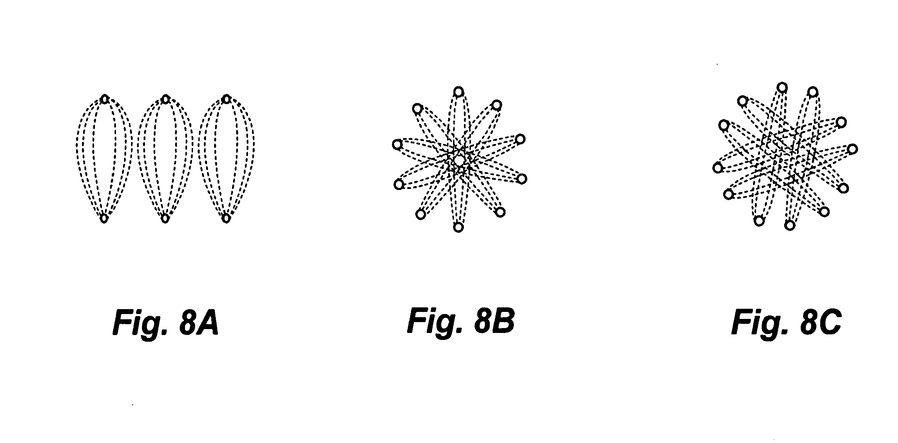

Figures **8A-C** are drawings showing prior art arrays with typically relatively uniform lines of force and corresponding electric fields between electrode array needles, as opposed to that of the invention wherein a non-uniform lines of force and respective electric field surrounds the array and dissipates rapidly therefrom. For example, Figure **8A** shows three opposing electrodes in a linear array wherein the lines of force between the electrodes are relatively uniform. In Figures **8B** and **C** is depicted circular arrays wherein the treatment zone is central to the electrodes and under relatively uniform lines of force and respective electric fields (individually pulsed in opposing pairs, Figure **8B****,** or pulsed in pairs of opposing electrodes in different orientations, Figure **8C****.**

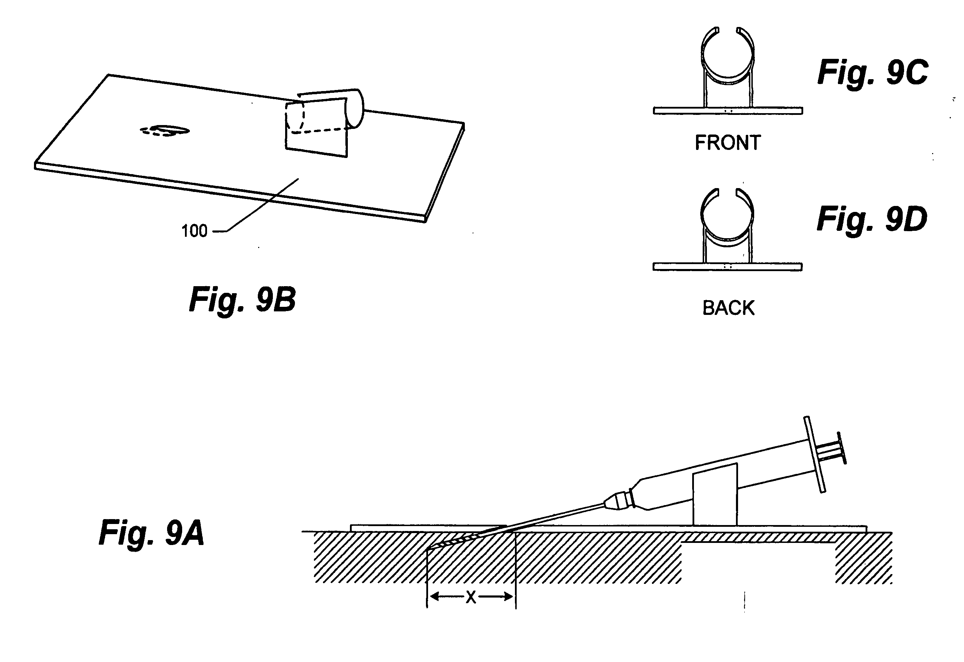

Figures **9A-D** show yet a further embodiment of the invention device which comprises a guide for resting the needle and reservoir for penetration of tissue to be treated at an acute angle for use in methods that include delivery of treatment substances near the tissue surface. This angle is typically between 3 and 25 degrees from the plane formed by the general area of the tissue surface.
Figures **10A and 10B** show partial view of delivery needles comprising electrodes exposed near the tip of the delivery needle. Figure **10A** depicts a needle supporting straight electrodes while Figure **10B** depicts a needle supporting spiral electrodes. The leads for each of the cathode and anodes are depicted running up an internal section of the needle. Also, this depiction is intended to represent that the upper portion of the elongate needles can comprise insulation either around the electrode leads and/or coating the upper needle shaft.

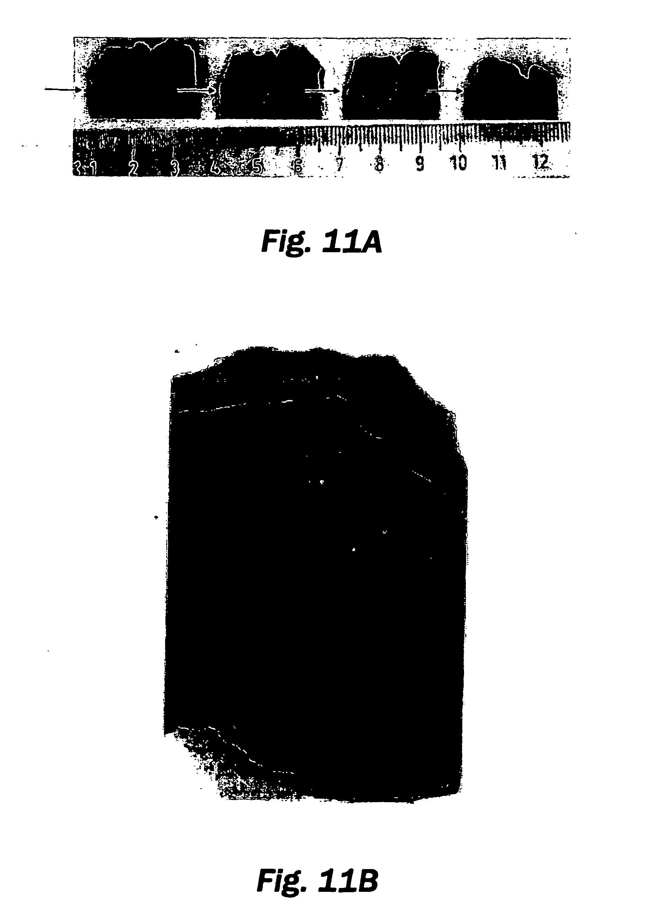

Figures **11A** and **B** show results of electroporation in a tissue wherein cells primarily near the needle track have been affected by electroporation. In Figure **11A** is a series of photos showing adjacent slices of tissue while Figure **11B** shows a close-up of a central slice directly along the needle track.

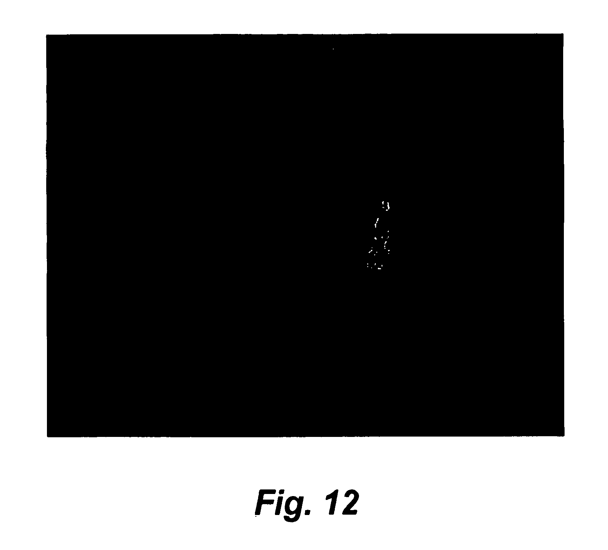

Figure **12** shows the results of a single injection into rabbit thigh muscle of a nucleic acid containing an expression vector encoding a fluorescent marker protein (GFP) using an electroporation device according to the invention.
Figures **13A****, B, C, D,** and **E** show magnified photographs of a prototype hypodermic needle wherein gold elongate electrodes have been etched onto a standard hypodermic injection needle using MEMS technology, i.e., micro layering, and etching and relayering of materials onto the base injection needle shaft such that the anode and cathode comprise 1/4 of the needle shaft circumference each. Figure **13** **A** shows one view of the needle showing one long electrode lead running the length of the needle. In Figure **13** **B,** a detail photo is shown from an angle allowing visualization of the terminal sections of both gold anode and cathode. Figure **13** **C** is another perspective showing detail of the terminal sections of the anode and cathode etched onto the needle shaft. Figures **13D** and **E** show another embodiment wherein the MEMs crafted anode and cathode are 1/16 the circumference of the needle shaft.
Figures **14** **A, B,** and **C** are drawings showing additional embodiments of single-needle design where in the shaft comprises electrically inert material such as for example, plastic extruded with electrode leads built into the extruded hypodermic shaft. Figure **14A** depicts straight anode and cathode running parallel to the needle shaft. Figure **14B** depicts the anode and cathode in a spiral about the shaft. Figure **14C** depicts the cross section AA-AA of Figure **14A** showing one embodiment wherein the anode and cathode of the shaft can be connected to electric leads positioned on the needle hub.

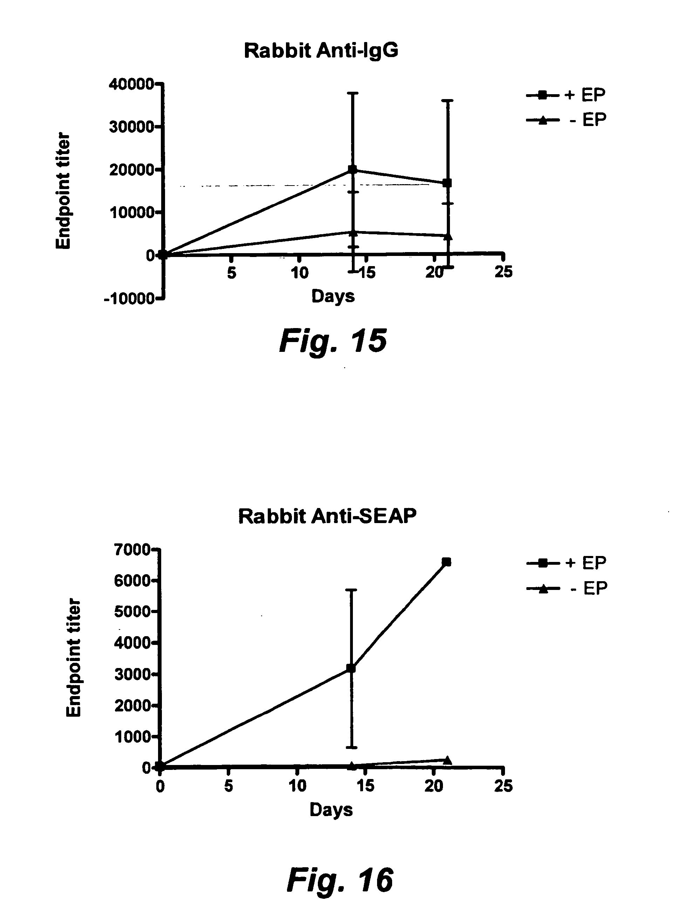

Figure **15** is a graph showing the level of rabbit anti-human IgG antibodies produced following electroporation pulse using the single-needle invention (■) versus no electroporation (▲).
Figure 16 is a graph showing the level of rabbit anti-SEAP antibodies produced following electroporation pulse using the single-needle invention (■) versus no electroporation (▲).

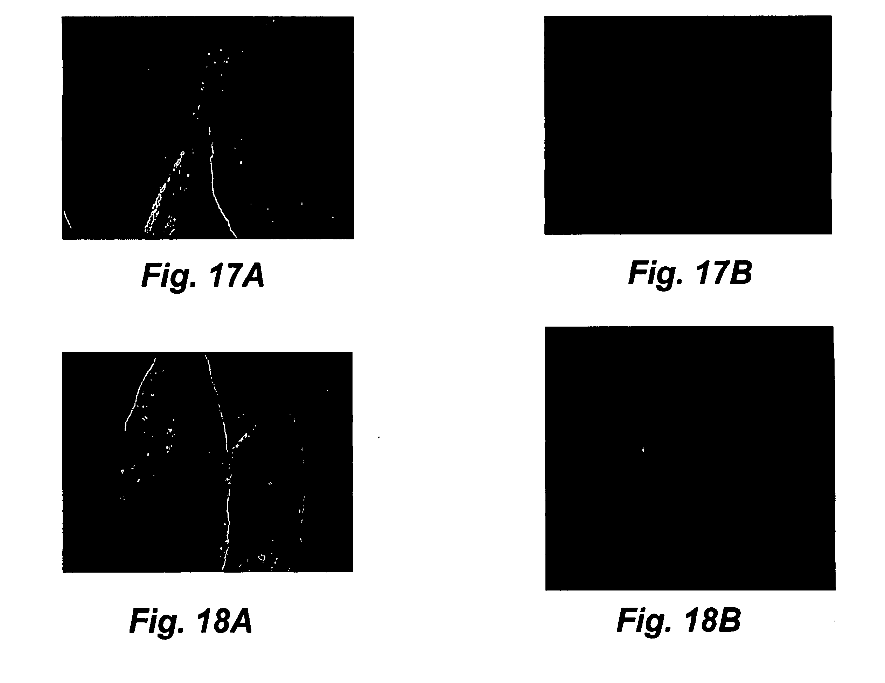

Figures **17**A and **B** are photographs showing results of green florescent protein (GFP) expression following injection of plasmid DNA encoding GFP followed by no electroporation. In combination of natural and fluorescent light, Figure 17A shows adjacent slices of tissue in the vicinity of the injection/needle track site. The photos show no expression without electroporation.
Figures **18A** and **B** are photographs showing combination of natural light and green florescence, or fluorescence alone respectively, wherein injection of plasmid DNA encoding GFP was followed by electroporation carried out using a single-needle electrode comprising a 23 gauge needle and anode and cathodes having a width of 1/16 the circumference the needle shaft. In this experiment, the electrodes were pulsed at a constant current of 50 mA.

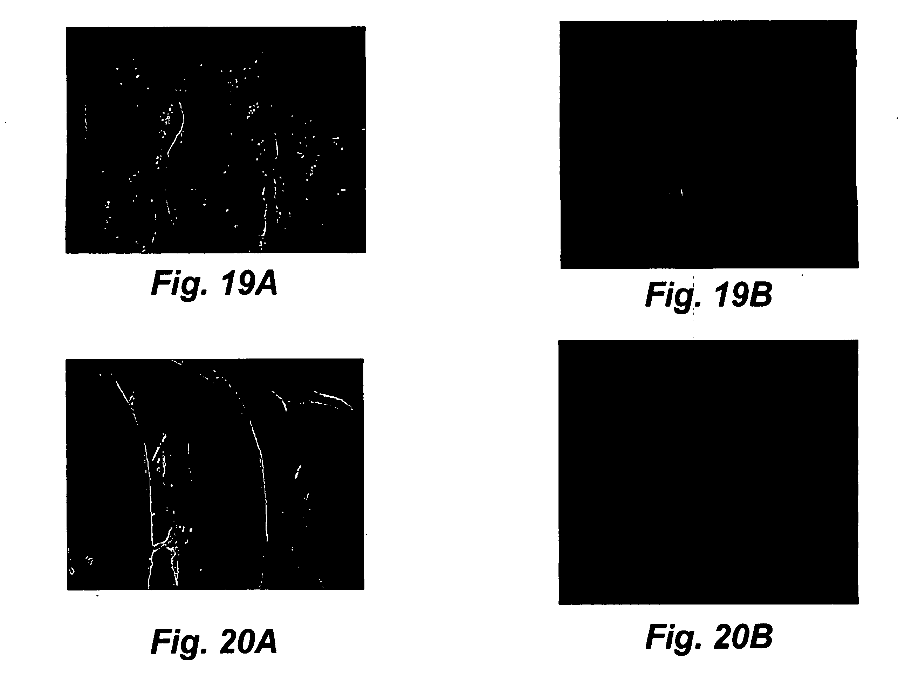

Figures **19A** and **B** are photographs showing combination of natural light and green florescence or fluorescence only, wherein injection of plasmid DNA encoding GFP was followed by electroporation carried out using a single-needle electrode comprising a 23 gauge needle and anode and cathodes having a width of 1/16 the circumference the needle shaft. In this experiment, the electrodes were pulsed at a constant current of 100 mA.
Figures **20A** and **B** are photographs showing combination of natural light and green florescence or fluorescence only, wherein injection of plasmid DNA encoding GFP was followed by electroporation carried out using a single-needle electrode comprising a 23 gauge needle and anode and cathodes having a width of 1/4 the circumference the needle shaft. In this experiment, the electrodes were pulsed at a constant current of 50 mA.

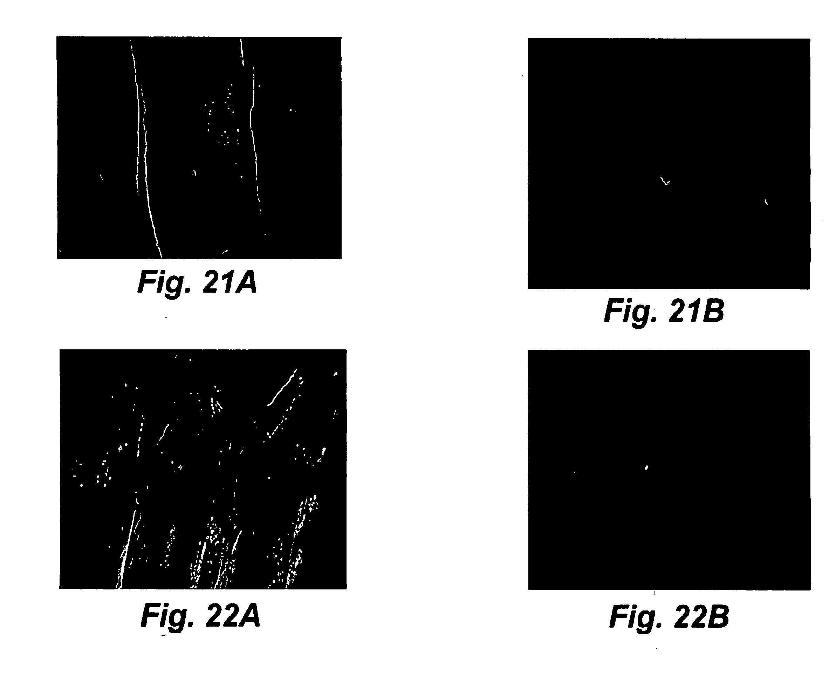

Figures **21A** and **B** are photographs showing combination of natural light and green florescence or fluorescence only, wherein injection of plasmid DNA encoding GFP was followed by electroporation was carried out using a single-needle electrode comprising a 23 gauge needle and anode and cathodes having a width of 1/4 the circumference the needle shaft. In this experiment, the electrodes were pulsed at a constant current of 100 mA.
Figures **22A** and **B** are photographs showing combination of natural light and green florescence or fluorescence only, wherein injection of plasmid DNA encoding GFP was followed by electroporation was carried out using a single-needle electrode comprising a 23 gauge needle and anode and cathodes having a width of 1/4 the circumference the needle shaft. In this experiment, the electrodes were pulsed at a constant current of 150 mA.

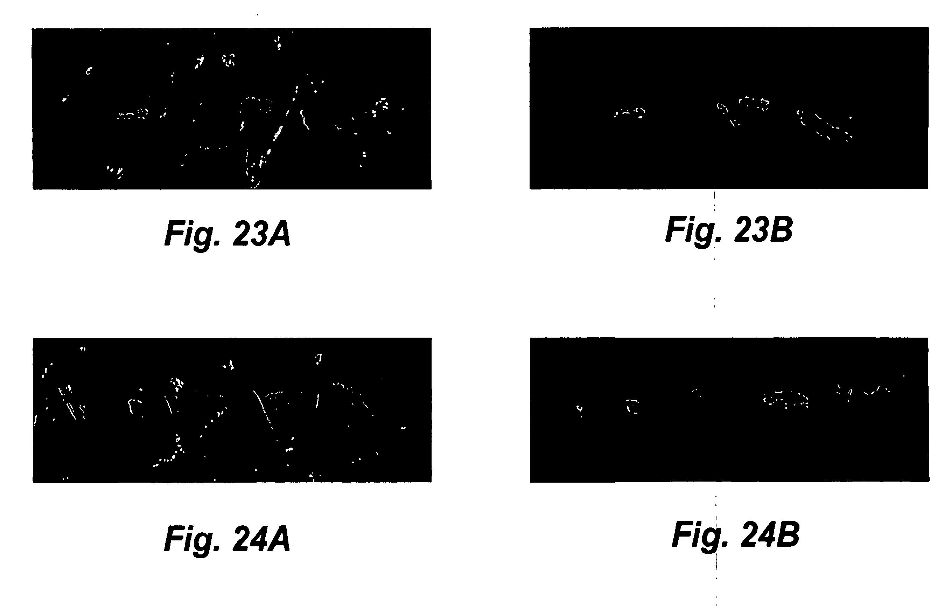

Figures **23A** and **B** are photographs showing combination of natural light and green florescence or fluorescence only, respectively, wherein injection of plasmid DNA encoding GFP was followed by electroporation was carried out using a single-needle electrode comprising electrodes 1mm spacing without fluid delivery embodiment and without any inert matter lying therebetween. In this experiment, the electrodes were pulsed at a constant current of 75 mA.
Figures **24A** and **B** are photographs showing combination of natural light and green florescence or fluorescence only, respectively, wherein injection of plasmid DNA encoding GFP was followed by electroporation was carried out using a single-needle electrode comprising electrodes 1mm spacing without fluid delivery embodiment and without any inert matter lying therebetween. In this experiment, the electrodes were pulsed at a constant current of 150 mA.

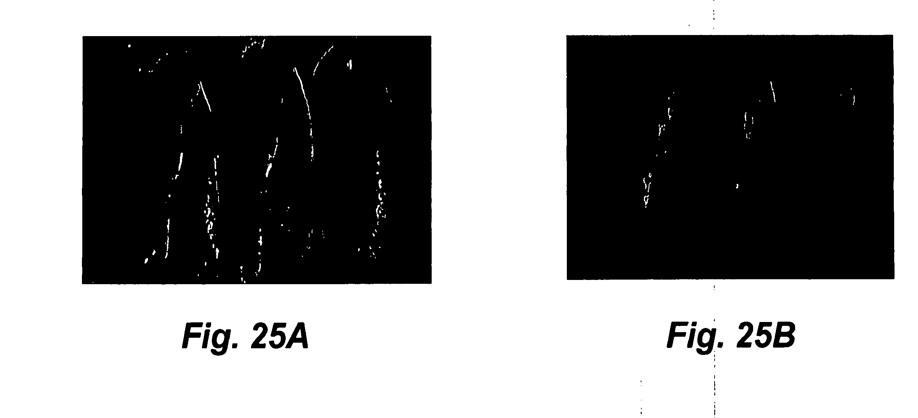

Figures **25A** and **B** are photographs showing combination of natural light and green florescence or fluorescence only, respectively, wherein injection of plasmid DNA encoding GFP was followed by electroporation was carried out using a single-needle electrode comprising electrodes 1mm spacing without fluid delivery embodiment and without any inert matter lying therebetween. In this experiment, the electrodes were pulsed at a constant current of 250 mA.

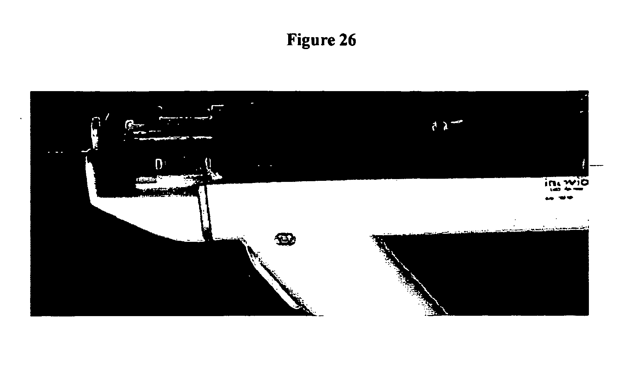

Figure **26** is a color photo of an Elgen electroporation device (Inovio, AS, Norway) equipped with a single-needle electrode.

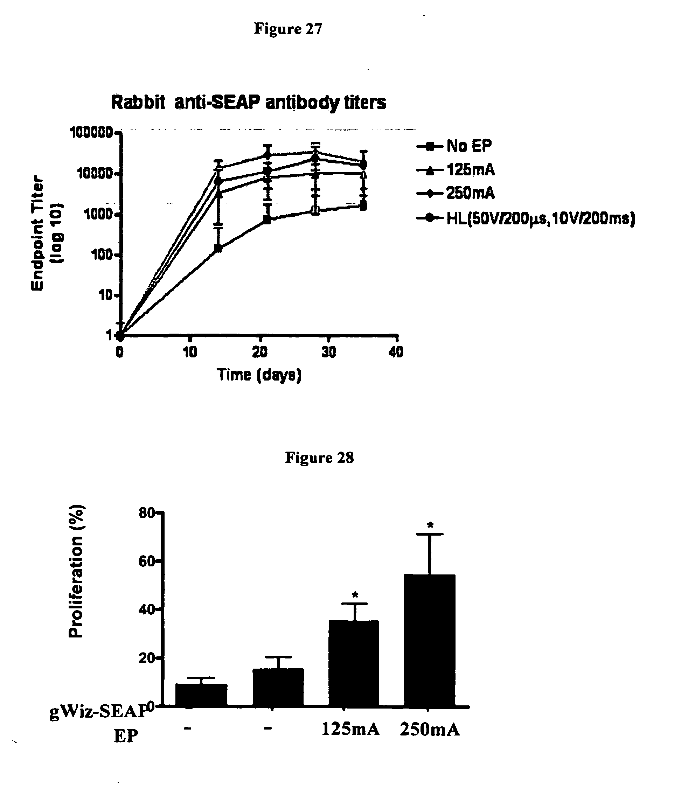

Figures **27** shows a graph of titer results showing enhanced IgG anti-SEAP titer expression in groups receiving electroporation using the single-needle electrode.
Figure **28** is a graph showing antigen specific cellular immune responses in rabbits using the single-needle device. In the graph rabbit PBMC proliferation percentage against PHA 8 ug/ml (100%) are provided.

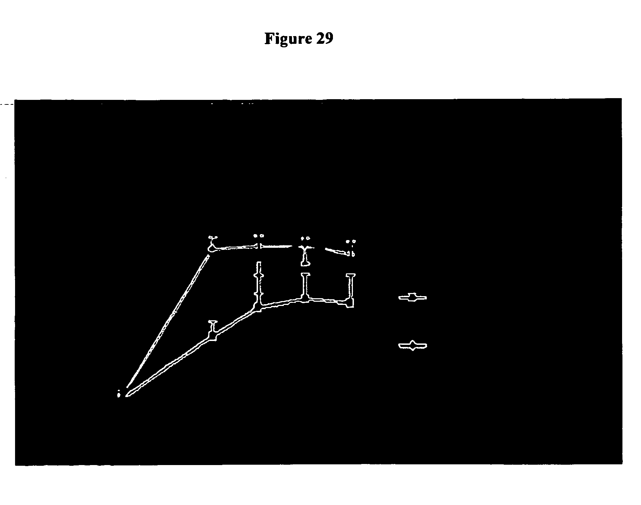

Figure **29** is a graph showing elicitation of rabbit IgG anti- hIgG3 antibody titer production using pulsing at two different voltages. * indicates p<0.05.

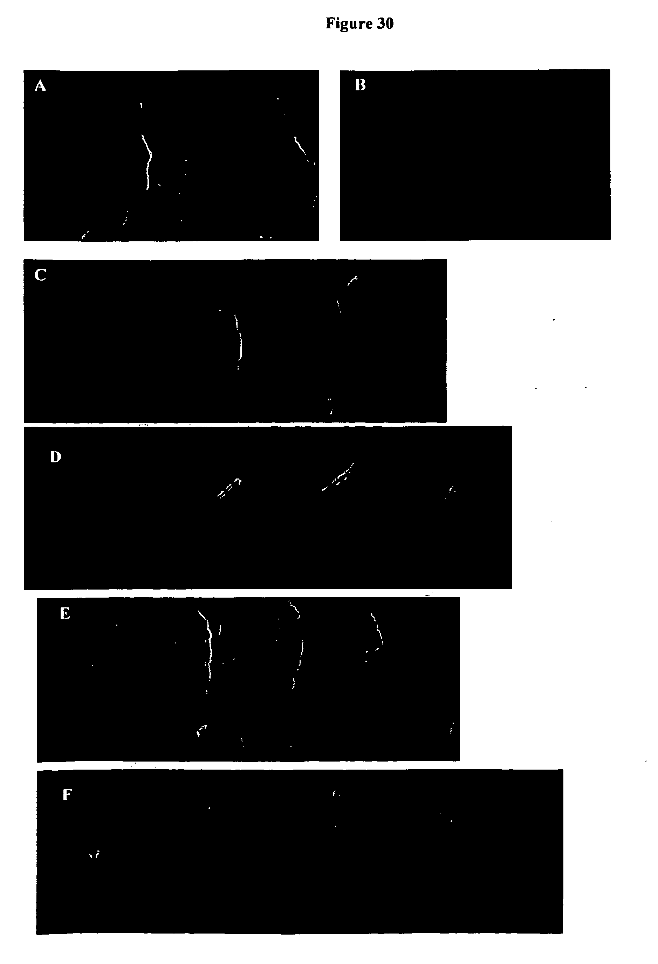

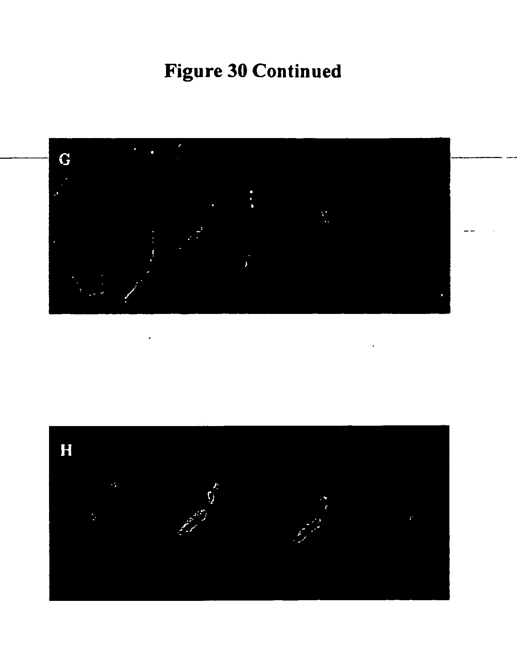

Figure **30A** to **H** are color photographs showing tissue slices of the injection sites of the single-needle electrode. In Figures **30**A and **B** is shown light field and fluorescence, respectively, without electroporation. In Figures **30** **C** and **D** are shown light field and fluorescence, respectively, wherein electroporation pulses used 8 V. Figures **30** **E** and **F** show light field and fluorescence, respectively, wherein electroporation pulses used 15 V, and Figures **30** **G** and **H** show light field and fluorescence, respectively, wherein electroporation pulses used 25 V pulses.

### DETAILED DESCRIPTION OF THE PREFERRED EMBODIMENTS

In a first embodiment, the invention comprises a device for electroporation of tissue *in vivo* comprising a hollow shaft made of a material capable of insertion into a biologic tissue or organ *in situ* and of delivering therethrough a fluid medium (i.e., a delivery needle shaft), said shaft further comprising at least two electric leads exposed at least in part on an outer surface of said shaft, wherein said electric leads are spaced from one another and situated parallel with respect to one another along said needle shaft. Embodiments for placement of the electric leads can employ a variety of structural designs. For example, anode and cathode electric leads can be placed in association with a delivery needle that run parallel to one-another and to the length of the delivery needle such as disclosed in Figures **1** and **3****,** or that are parallel to each other but are spiraled around the needle shaft as depicted in Figures **2** and **4****.** The invention device also includes electric conduits connecting each of said anode(s) and cathode(s) to an electrical energy source wherein said electric conduits when said needle is inserted into a patient tissue are capable of being energized individually, generating an electric field to cells in a treatment zone surrounding said needle shaft sufficient to cause cells along and near a track made by insertion of said needle into said tissue to become reversibly porated so as to allow treatment molecules to enter said cells. To facilitate the delivery of substances along the tissues of the needle track made by insertion of the delivery needle, the needle shaft comprises a fenestrated needle having therein delivery ports along the length of the needle, or at least a portion of the length of the needle, in addition to or alternatively in place of a delivery port at the distal end of the needle shaft.

Manufacture of such single-needle electrode containing fluid delivery needles can be carried out by any number of well know methods including micromachining such as commonly understood as MEMS technology. For example, a standard hypodermic needle (which can be any gauge such as 20gauge, 21 gauge, 22 gauge, 23 gauge, 24 gauge, 25 gauge 26 gauge, 27 gauge, 28 gauge and 29 gauge) can be coated with an electrically inert material followed by deposition of electrically conductive material such as gold, followed in turn by etching away the conductive material in the orientation desired on the surface of the needle. Specifically, generally the process comprises cleaning the hypodermic needle shaft in preparation for deposition of the inert substance, for example, a polymer having properties of evenly adhering to surfaces, such as parylene. Following stripping of the metal shaft, parylene is deposited, such as by vacuum deposition, on to the needle. This is in turn patterned using a laser to deposit electrode conductable material, such as gold, followed in turn by selective removal of the gold to form anode and cathode electric leads in a predetermined pattern on the needle shaft. In the current invention, the use of MEMS technology provides for an ability to manipulate the three dimensional needle and coatings and etchings on a miniature scale. The capability to manufacture a single-needle electrode is proven by the photographs of Figures **13A** to **E****.** Manufacture can also be carried out by extrusion technology. As depicted in Figures **14A-C****,** in this aspect the electrodes **202** and **203** (Fig. **14A**) are extruded as fine wire filaments with an electrically inert substance such as polyvinylchlorine or the like in a linear fashion. The tip of the needle **204** can be machined or cut to a tissue penetrating tip and at the other end fitted to a hub **200** comprising electrode leads **201a** and **201b** and a fitting **205** for attachment to a source of fluid medium. Figure **14B** depicts an example of a structural embodiment comprising an extruded needle with spiral electrodes and electrode leads 210 and 211.

The present disclosure provides a method for delivering molecules to cells *in vivo* comprising providing to a patient's tissue containing said cells an injection needle further comprising at least two elongate electric leads (i.e., a cathode and an anode) positioned along the needle shaft and at least a reservoir containing said molecules wherein said reservoir and molecules are in fluid communication with a lumen running through said needle shaft, injecting the molecules into said tissue, and energizing the electric leads with electrical energy to provide an electric pulse sufficient to cause cells in the vicinity of the injection site and needle track to become reversibly porated, thereby electroporating said cells for their uptake of said molecules.

In an embodiment, the device provides for electroporation of cells in a narrowly defined location, particularly cells along or near the track make by the single-needle electrode device. Generally, the cells considered within the treatment site are those cells lying in a radius around the needle track of about 6 to 5mm, more typically about 3mm, and even more particularly about 2mm, and most particularly about 1mm. In a related embodiment, the generation of electric fields sufficient for electroporation of cells comprising said treatment site is a field that weakens outward from the centrally located single-needle electrode insertion site such that the treatment site is defined by the inability of the pulse energy to extend into the tissues beyond a certain distance from the single-needle electrode.

In a further related embodiment, the invention calls for the novel use of a single elongate probe, which comprises the injection needle and anode(s) and cathode(s), for performing *in situ* electroporation of a highly localized set of cells in the tissue.

In another embodiment, the invention device may be used with any of a variety of electric pulsing conditions. For example, the single-needle electrode can be charged with at least one pulse of constant current in the range of between 1-1000 mAmps, typically between 10-500 mAmps, and more preferably between 20 and 250 mAmps.. In another example, the electrodes can be charged with a voltage pulse in the range of 1 to 100 volts. Further, the electric pulse can be either a monopolar or a bipolar pulse wherein said pulse can be a single, a double or a multiple pulse sequence having various characteristics such as a set voltage drop, variable shaped pulse trains, or pulses employing constant current.

In other embodiments, the device and method provide for delivering or transfecting pharmaceutical drugs, proteins, nucleic acids including DNA and RNA, and synthetic modifications thereof, including RNAi, as are well known to those of skill in the art, into patient tissues, particular to cells residing in the subcutaneous, intradermal, and subdermal spaces and as well as skeletal and striated muscle compartments of a mammalian body, and organs including heart, lung, pancreas, spleen, liver, and organs of the alimentary tract. Once transfected with the selected material, cells will be directly affected by the activity of the drug, or protein or nucleic acid. Where nucleic acids are transfected, typically such nucleic acids are employed for the protein encoded thereby that can be expressed in the cells of the treatment site. Further, the substances can comprise cytokines, chemokines, and immune relevant bioactive molecules including such active molecules as immune modulating molecules selected from the group consisting of IL-1, IL-2, IL-3, IL-4, IL-5, IL-6, IL-7, IL-8, IL-9, IL-10, IL-11, IL-12, GM-CSF, M-CSF, G-CSF, LIF, LT, TGF-E, IFN, TNF-D, BCGF, CD2, or ICAM.

In another embodiment, the material to be delivered to the cells can be delivered in a liquid form in a volume of between 0.0 1 ml to 1ml. In one embodiment, nucleic acid encoding a polypeptide can be dissolved in 0.9% sodium chloride (NaCl). The exact solvent, however, is not critical to the invention. For example, it is well known in the art that other solvents such as sucrose are capable of increasing nucleic acid uptake in skeletal muscle. In a related embodiment, the volume to be delivered can be adjusted in relation to the length of the needle (since the length of the needle shaft will determine both the volume of the substance being transported therethrough) and, the needle track made so as to determine the volume of the space available for said substance to fill upon it being expressed through the needle and into the needle track and surrounding tissue. For example, a 2mm long needle can be used for delivering substances to skin layer tissues and provide for injection of a volume in the range of 0.01 ml to 0.05ml, while a 5mm long needle can be used to deliver volumes in the range of 0.1ml to 0.15ml, and a 1.5 to 2 cm long needle can be used for delivering volumes in the range of 0.3ml to 0.5ml.

Other substances may also be co-transfected with the molecule of interest for a variety of beneficial reasons. For example, the molecule P199 (lee, et al. PNAS., 4524-8, 10, 89 (1992)), which is known to seal electropermeabilized membranes, can beneficially affect transfection efficiencies by increasing the survival rate of transfected muscle fibers.

With reference to Figure **6****,** for example, the single-needle electrode is inserted into a patient tissue to a desired depth of penetration. The plunger of the attached syringe is activated to inject the volume of liquid containing the selected material for injection, and the electric leads (i.e., anode(s) and cathode(s)) are immediately thereafter, or alternatively simultaneously with the injection of the material, energized with at least one pulse of electric energy sufficient to cause at least some of the cells in the treatment zone to become reversibly porated. Although the syringe plunger is typically activated using animate means, such as by use of the hand, the syringe can also be affixed to a holding device such as disclosed in Figure **9****,** or even an automatic dispensing apparatus, such as a device disclosed in US patent application US 2004/0059285 filed July 3, 2003 (See Figure **26**).

In other embodiments, the invention can be applied to electroporation of cells at various depths from the surface of a body tissue. For example, besides electroporation of cells residing within muscle tissue compartments in which delivery of substances are initiated by injection of materials into the tissue in an orientation approximating 90 degrees from the surface of the tissue, in one embodiment the invention device can be used to electroporate cells in the subcutaneous, intradermal, or subdermal spaces of skin. It can also be used to electroporate substances into lymph nodes, or tissue layers in other organs such as cardiac and blood vessel tissue. With respect to electroporating cells in any of these locals, use of the device for electroporating cells in such tissue layers can include use of either short needles having a length sufficient for penetrating outer portions of the tissue layers (i.e., skin, subdermal, etc.) for injection and electroporation at approximately a 90 degree angle to the tissue surface, or where a delivery needle is relatively long, such as between 3 and 4 cm, insertion of the single-needle electrode can be made at an acute angle to the surface tissue using a holding device as depicted in Figure **9A****.** This will allow for electroporation of a larger portion of tissue within the desired layer. Further, the acute angle of insertion can be between 3 to 25 degrees of angle from the tissue surface. Such tissue surface can be described as forming generally a flat surface area forming a plane encompassing the site for insertion of the single-needle electrode. As depicted in Figure **9A** to **D****,** the syringe can be connected to an attachment means which is designed to hold the syringe at a set angle on a planar guide tray **100** with the needle placed a set distance ***X*** into the tissue as determined based on the predetermined desired depth of insertion of the needle into the tissue. The guide tray, with exposed needle, is brought into contact with the tissue surface such that the needle inserts the tissue at the prescribed acute angle. After the needle is so inserted and the therapeutic substance expelled from the syringe, the electric leads of the single-needle electrode can be energized to bring about delivery of the injected material into the subcutaneous, intradermal, or subdermal cells. Use of the device at an oblique angle as discussed above can also apply to electroporating various layers of organ tissue.

### Examples:

The following examples are given to illustrate various embodiments that have been made of the present invention. It is to be understood that the following examples are not comprehensive or exhaustive of the many types of embodiments that can be prepared in accordance with the present invention.

### Example I.

Turning now to various aspects of the invention, the device can comprise molecule delivery reservoir **20** and single-needle electrode **10** components as shown, for example, in (Figure **5**). Additional embodiments include sharps cover **11,** resilient membrane **12** sealing a portion of the structure comprising the reservoir **20** for uses in filling the reservoir (such as by piercing of a syringe needle), and mechanisms such as dimples **13** and recesses **14** and **14*** in the reservoir **20** housing structure for keeping the sharps cover **11** in a semi-fixed position of either open/retracted (Figure 5C), or closed/covered (Figures **5A** and **B**). Further embodiments include mechanisms for keeping the plunger **9** in a semi-fixed open/retracted or a closed/expelled position, such as, for example, dimples **15** and recesses **16** and **16***. It should be clear to one of skill in the art that regardless of the method employed to provide for semi-fixed positioning of the sharps cover **11** and plunger **9,** such positioning can easily be changed with either animate energy, such as force by hand, or mechanically, such as by an electronically driven actuator. The distal end of the sharps cover **11** can include removably attached thereto a sterility cover **60.** The single-needle electrode needle **10** further can comprise a lumen running therethrough ending in tissue piercing tip **22,** and orifice **25** for connecting to the reservoir **20** (See Figure **1****).** The single-needle electrode needle 10 can be of a gage between 18 and 29 standard hypodermic needle gauge sizes. In a preferred embodiment, the single-needle electrode comprises at least one pair of electric leads, such as leads **21a** and **21b** of Figure **1****.** The electric leads comprise at least one anode and one cathode which are in electrical communication with electric leads **24a** and **24b.** Depending upon the design chosen for any particular invention product, the leads can terminate in a lead terminal **23** (see Figures **3** and **4****,** for example), or connect by any other means with lead wires running from the single-needle electrode to a source of electrical energy, such as a pulse generator. The needle component **10** can further include a connector **26** (Figures **3** and **4****)** for attaching to a hypodermic syringe reservoir, or to a syringe reservoir affixed with a locking mechanism to detachably fasten the needle component **10** to a hypodermic syringe port.

In further embodiments, the reservoir **20** can be manufactured with a predetermined substance for treating a particular condition. Alternatively, the reservoir can be filled with a substance of interest by either drawing such substance into the reservoir through the electrode needle **10** by extracting the plunger **9,** or preferably, the reservoir can first be cleared of the plunger by retracting the plunger to the open position followed by delivering to the reservoir the substance by injecting it into the reservoir via the resilient seal **12,** similarly to the procedure commonly performed in the extracting of drugs from sterile vials into syringes and introducing them into another reservoir.

The single needle electrode **10** with its array of anode(s) and cathode(s) (such as electric leads **21a** and **b, 31a** and **b, 51a** and **b** and **52a** and **b,** or **41** and **42,** Figures **1-****4,** respectively) can be inserted into the tissue, usually at an approximate 90 degrees to the tissue surface, or alternatively at an acute angle with respect to the tissue surface, and the substance injected into the needle track and local tissues. The single-needle electrode can be energized using a pulse generator either following the injection of said substance, or can be energized simultaneously with said injection of substance. As depicted in Figure **6****,** when energized with an electric pulse, the single-needle electrode supports the generation of an electric field **20** that provides for sufficient energy to cause reversible poration of the cells within said field. The electric filed generated is non-uniform in that it is believed to exponentially decrease by the distance from the needle track **80** (Figure **7****).** Thus, the electric field sufficient to provide such poration has, depending upon the energy employed, symmetrical lateral dimensions (a) x (b) (shown in Figure **7****)** forming a set diameter of an electroporating electric field which, with respect to the needle track length, forms a defined three dimensional volume. Generally, the poration-sufficient electric field has a radius from the single-needle electrode needle **10** of between 0mm and 5 mm and even 6mm, typically between 0mm and 4 mm, and preferably between 0mm and 3 mm and most preferably between 0mm and 2 mm.

As will be understood by those having skill in the electroporation arts, the field generated by the current invention's single-needle electrode, unlike prior electroporation apparatuses, is a non-uniform electric field wherein the field intensity is greater near the needle and diminishes as measured outward from the single-needle electrode. In contrast to the current electrode arrangement, Figure **8** depicts prior electrode arrangements wherein a uniform electric filed is employed across a large volume treatment site. The instant invention is measurably distinct from former concepts that suggested a need to utilize a "uniform" field. Here, the invention employs a non-uniform field which provides for reversible poration of cells to a greater amount near the position of the delivery needle, i.e., the needle tract. This in turn allows a clear benefit to determine the precise location of those cells receiving a known dose of therapeutic materials. This invention through its embodiments therefore provides for "fitting" the electric field to the injection site so as to distribute material to cells more uniformly and confined to a local tissue area as opposed to the variable distribution allowed for with electroporation systems that use a conventional uniform electric field and an outer array of electrodes.

With respect to the electric leads generally, they can comprise any metal but preferably are a metal that does not impart a toxicity due to metal ions to the cells of the electroporated tissue. Such materials include gold, tungsten, titanium nitride, platinum, platinum iridium, and iridium oxide. The electrode material can be formed on the delivery tube (i.e., injection needle) such that there is a layer of insulation between the electrodes and the delivery tube as suggested in Figure **3****B.** Alternatively, the needle can comprise a material that is nonconductive itself eliminating a specific need to insulate the electric leads from the injection tube. In this aspect, the delivery tube can be constructed from any suitable material for insertion into tissue *in situ* that is nonconductive, including, such as a ceramic, or hardened biocompatible plastic, including polyvinylchlorine or the like.

In a further embodiment, the delivery needle/electrode component can be designed such that the electric leads **90** or **101** (Figure **10****)** are exposed for electroporation only near the tip of the needle as depicted in Figures **9A****,** and **10A** and **B****.** The unexposed portions **91** and **102** of the electric leads can be insulated and run along the delivery needle exterior or internal to the needle. Specifically, where it is desired to position the defined treatment volume (defined by the dimensions of the electroporation electric field imparted to the tissue by the array of electric leads) in a particular tissue, with the intent of avoiding electroporation of other tissues, electric leads, such as disclosed in Figure **10****,** can be used, for example, to electroporate deep muscle tissue and avoid other tissues lying closer to the tissue surface, such as fat cell layers, or alternatively to electroporate tissues near the surface, such as for example, subdermal tissues, as suggested in Figure **9A****.** Such embodiments provide for additional control over placement and size of the treatment volume.

### Example II

In this example, results are depicted for delivering molecules by reversible poration to cells situated along and near the track formed by the insertion of the invention single-needle electrode into a tissue.

As depicted in Figures **11A** and **B****,** rabbit quadriceps muscle was injected with DNA encoding beta-galactosidase in a bolus comprising 0.2 ml and DNA concentration of 1 mg/ml. The electrodes were pulsed using 2 pulses of 250 mAmps, 20 millisec duration. Following electroporation, the beta-galactosidase gene was expressed in cells affected by the electroporation. At day 4 after electroporation, the rabbits were sacrificed and the muscles were prepared in 3 mm thick slices through the site on insertion of the single-needle electrode. Following chemical fixation, the beta galactosidase expressing cells in the muscle slices where visualized by an enzymatic reaction. The arrows in Figure **11A** depict the direction of the insertion of the electrode into the rabbit muscle. As shown, staining occurs predominantly along the track formed by insertion into the tissue of the electrode.

### Example III

This example describes experiments that employ an electroporation device according to embodiments of the invention to deliver DNA encoding green fluorescent protein (GFP) into rabbit quadriceps muscle. The results are shown in Figure **12****.**

New Zealand white male rabbits, each weighing 4-5 kg (Perry Scientific, San Diego, California), were each injected with an expression vector (gWizGFP, lot 12311, purchased from Aldevron, LLC, Fargo, ND; see also Gene Therapy Systems, Inc., San Diego, CA) encoding a bright GFP (Cheng, et al. (1996), Nature biotechnology, vol. 14:606-9) the expression of which was under the control of a modified human cytomegalovirus immediate early promoter/enhancer.

Prior to injection, each rabbit was first sedated with acepromazine (1mg/kg) and then anesthetized by intramuscular injection of a mixture of ketamine (35 mg/kg) and xylazine (5 mg/kg) in the presence of glycopyrrolate (0.01 mg/kg), which had been previously administered subcutaneously to prevent uneven heart beating as a result of the ketamine/xylazine treatment. The rabbit was then shaved at the site where the injection was to be made, i.e., into the quadricepts muscle. A hole was poked in the skin covering the muscle by first inserting an 18 gauge needle, and then slightly widened using a scalpel. A single-needle electroporation device comprising an 18 gauge hypodermic needle with two parallel electric leads (anode and cathode) applied opposite one another to the outer surface of the needle (as depicted in Figure 1), was then slowly inserted into the muscle tissue, periodically pausing to inject DNA every few millimeters to a final insertion depth of approximately 25 mm. A total of 500 ul of DNA-containing solution containing 100 ug gWizGFP was injected into each injection site. Shortly after completing the injection and while the single-needle electrode device was still inserted to its final insertion depth, electroporation was commenced. Specifically, five 250 mA pulses, each of twenty millisecond (ms) duration, were applied to the electroporation needle device at 10Hz intervals (i.e., 100 ms) using an Elgen 1000 (Inovio AS, Oslo, Norway) current-clamped pulse.

Four days post-treatment the animals were humanely euthanized. Skin covering the region of the leg where the vector was delivered was carefully removed, after which each animal was placed at -20°C for about 1 hour. Treated muscle was then removed using a scalpel and then placed at -20°C for another 1 to 2 hrs. The frozen muscle tissue was then sectioned into slices approximately 3 mm thick using a rotating meat slicer. Muscle slices where arranged in plastic trays and examined for GFP expression using a Leica MZ 12 dissection microscope fitted with a UV light and GFP filter combination. Figure 12 is a photo representative of the results obtained by this analysis, and clearly shows that an electroporation device according to the invention can be used to successfully deliver an agent, for example an nucleic acid based expression vector encoding a desired protein that is then expressed in active form, into cells.

### Example IV

In this example, data for which is shown in Figures **15** and **16****,** using the invention electrode configuration, plasmids encoding SEAP (pSEAP#3 348, Aldevron) and IgG (pLNOH 2hg3 #11765, Aldevron) were electroporated into cells of test animal tissues (i.e., intramuscular injection into the tibialis anterior of the animal) and the expression monitored to prove success of expression in rabbit muscle as well as measuring immune responses against both a "week' and a 'strong" antigen (SEAP and IgG, respectively). In these experiments the SEAP and IgG plasmid were administered at a final concentration of 1 ug/ul.

Animals used were New Zealand White male rabbits 3.5 to 4.5 kg. Electroporation was carried out using an Elgen 1000 (Inovio AS, Oslo, Norway Serial number 009) which further comprised a current-clamped pulse generator (prototype) and a single-needle electrode wherein the electric leads (anode and cathode) ran parallel to the length of the needle shaft and spaced, due to the diameter of the needle of approximately 1 mm, apart. The single-needle electrode was pulsed for 20 millisec pulse length with 5 pulses each at 150 mA with a 250 millisec interval between pulses (i.e., a frequency of about 4Hz). The anode and cathode extended into the tissue to about 1.0 cm depth.

The experiments each comprised a two-step delivery process, i.e., injection of the plasmid solution (200 ul) using a 29 gauge insuline syringe with injection during insertion of the needle to distribute DNA at different depths, followed by removal of the injector needle and insertion of the single needle electrode.

As shown in Table **I** below, each of the IgG and SEAP experiments had two groups of test animals, i.e., one set of animals receiving electroporation and the other not (control)

**Table I**

| Group | Current | Treatment |
|---|---|---|
| | 150-250 | 100 ul x 2 SEAP 1mg/ml, 100 ul x 2 left tibialis, IgG 1 mg/ml |
| **1** | mA | 100 ul x 2 right tibialis |
| | No EP | 100 ul x 2 SEAP 1mg/ml, 100 ul x 2 left tibialis, IgG 1 mg/ml |
| **2** | | 100 ul x 2 right tibialis |

Samples were taken Day 0, 14 and day 21. The rabbits were then terminated on day 21 with subcutaneous injection of 0.5 ml hypnorm (Hypnorm 0.1 ml/kg) followed by i.v. injection of 1 ml/kg of 10% Phenobarbital in the ear vein.

As is clear from the results of Figures **15** and **16****,** the levels of antibody titer elicited from the single-needle electrode are far in excess of the negative control. Specifically, the two test antigens (IgG and SEAP) elicited titers relative to one another as expected with IgG being a much stronger antigen that SEAP (see titer scale). Both antigens elicited antibody production in the electroporated samples and virtually no antibody production in the non-electroporated samples.

### Example V

In this experiment, prototype MEMs manufactured single-needle electrodes were tested in rabbit tissue using a variety of pulsing energies and green florescent protein expression. As indicated in Table **II,** three different electrode embodiments were tested, (1) a single-needle electrode in which the anode and cathodes were applied to a 23 gauge needle at 1/16 the circumference of the needle each, and applied to the full length of the needle by MEMs technology (Figures **13D-E****),** (2) a single-needle electrode wherein the anode and cathode are 1/4^{th} the circumference of the needle shaft each (Figures 13A-C), and (3) a single-needle electrode wherein the anode and cathode are 1 mm apart without a fluid medium delivery tube or other material lying therebetween. As shown in Table **II,** the various combinations of pulsing were performed.

The protocol used for each animal in this experiment comprised injecting the GFP plasmid at the noted concentrations, electroporating the tissue using one of the three above embodiments of the single-needle electrode, followed by sacrificing of the animals and performing tissue preparation by slicing the treated muscle in adjacent slices and observing florescence. Generally, due to the difficulty of slicing the tissue so as to retrieve slices parallel to the injection track, GFP florescence in the figure photos often show up as circles or elipses. These florescence patterns prove that the single-needle electrode is functional and provides for electropration of tissue at very low voltages and relative electric currents in defined locations surrounding the needle track within the tissue.

**Table II**

| **Electrode design** | **Tissue site** | **Constant current** | **Voltage (average V)** | **Number of pulses** | **pGFP DNA concentration/volume** |
|---|---|---|---|---|---|
| Electrodes 1/4 shaft circumference | Quadriceps | 0.0 | 0.0 | 0.0 | 0.2 mg/ml |
| Electrodes 1/16 shaft circumference | Quadriceps | 50 mA | 8 | 2 | 0.2 mg/ml |
| | Quadriceps | 100 mA | 18 | 2 | 0.2 mg/ml |
| Electrodes 1/4 shaft circumference | Quadriceps | 50 mA | 11 | 2 | 0.2 mg/ml |
| | Quadriceps | 100 mA | 15 | 2 | 0.2 mg/ml |
| | Quadriceps | 150 mA | 20 | 2 | 0.2 mg/ml |
| | Quadriceps | 250 mA | 33 | 2 | 0.2 mg/ml |
| Electrodes 1mm spacing without fluid delivery embodiment | Tibialis | 75 mA | 13 | 2 | 1.0 mg/ml |
| | Tibialis | 150 mA | 18 | 2 | 1.0 mg/ml |
| | Tibialis | 250 mA | 28 | 2 | 1.0 mg/ml |
| | Quadriceps | 150-200 | 20 | 2 | 1.0 mg/ml |
| | Quadriceps | 25 0-500 | 40 | 2 | 1.0 mg/ml |
| | Quadriceps | 600-1000 mA | 50 | 2 | 1.0 mg/ml |

Figures **17A** and **B** show both natural light and florescent light, respectively, photographs of GFP expression following injection of plasmid DNA encoding GFP with no electroporation. As indicated, there is virtually no green florescent protein expression. Thus, it is clear that without electroporation there is not sufficient uptake and expression of the desired gene.

With respect to electroporation *in situ* using the 1/16 width spacing of the anode and cathode about the needle shaft, the ability to express electroporated GFP is shown in Figures 18A and B and 19A and B. Figures 18A and B show GFP expression results upon electroporation with a constant current of 50 mA, while Figures 19A and B show electroporation at 100 mA.

For GFP expression using the ¼ circumference single-needle electrode, results are provided in Figures 20A and B, 21A and B, and 22A and B, wherein electoporation was carried out using 50, 100, and 150 mA, respectively.

GFP expression was also testing using an embodiment wherein the single-needle electrode did not comprise a fluid delivery tube associated with the electrodes. As shown in Figures **23A** and **B, 24A** and **B****,** and **25A** and **B****,** this invention device embodiment was tested at 75, 150, and 250 mA each at constant current. Here, the amount of GFP plasmid was five times the concentration of the experiments shown in Figures **19-22****.** Consequently, the treatment zone appears more readily.

### Example VI

In this example, an experiment was designed similar to that in Example IV. However, both resulting antibody titers and cellular responses were evaluated back to back for data consistency.

In this experiment, the single-needle device comprised a standard 23 gauge x 38mm hypodermic injection needle. The needle anode and cathode were applied via MEMS technology with 1/4 separation from each other about the needle shaft. The single-needle device was mounted on a standard 1 ml syringe followed by injecting plasmid DNA intramuscularly prior to delivery of electrical pulses via the same single-needle electrode.

Plasmid constructs for the experiment were gWiz-SEAP, expressing secreted placental alkaline phosphatase (SEAP) under the human control of the CMV promoter/enhancer (obtained from Aldevron LLC, Fargo, ND).

In NZW male rabbits (3.0-4.0 Kg), quadriceps muscles were primed with the DNA and electroporated. Specifically each group comprised six animals each receiving one injection per prime. The animals were again boosted by a second electroporation treatment after 2 weeks. In each inoculation, 400ug of gWiz-SEAP in 200 ul saline was injected per site of treatment in each rabbit followed by electroporation through the same single-needle electrode. The electrical pulse in each instance of prime and boost comprised 2 pulses at constant current of 125 or 250mA which corresponds to approximately 15V or 25V, 60 ms pulse length, 250 ms interval between the pulses that gives about 4 Hz frequency. One group of animals received Electroporation using a combination of or high & low voltages (HL) in a pulse train i.e., 50V for 200microseconds followed by 10V for 200milliseconds.

Blood samples for anti-SEAP antibody detection were collected at 2, 3, 4, 5 weeks after the first immunization by ear artery bleeds. The expression of rabbit antibody titer (rabbit IgG anti-SEAP) in sera was analyzed by enzyme-linked immunoassays (ELISA) conducted as well described in the immunologic arts.

Results showed an enhanced anti-SEAP IgG serum titers following DNA vaccination with electroporation in rabbit quadriceps. Specifically, rabbits were primed and boosted in 2 weeks with gWiz-SEAP in the absence or presence of electroporation with 125mA, 250 mA or high & low voltages (HL, 50V/200microseconds & 10V/ 200 milliseconds). In Figure **27****,** data from individual rabbits were pooled from two identical experiments, with median value indicated by a horizontal bar. The time course of anti-SEAP serum titers are shown for groups receiving no electroporation, electroporation of 125mA, electroporation of 250mA, and group using the high and low voltage pulses. The antibody titers are significantly higher than that of DNA injection without electroporation (*indicates p<0.05).

EP-enhanced cellular immune response in rabbits using a single-needle electrode was also shown as exhibited in Figure **28****.**

For measuring cellular immune responses blood samples were collected 2 weeks after boosting in sodium heparinized collection tubes (BD), and PBMCs were isolated by density gradient centrifugation using Histopaque-1077 (Sigma) and then cultured at 2x10⁵ cells/well in 96-well plates using RPMI-1640-complete for 3 days while stimulated with 2.5 µg/ml of SEAP. During At the end of culture, 25 ul of Celltiter 96 Aqueous reagent (Promega) was added to each well and incubated for 2-4hrs, followed by reading the absorbance at 490nm. Assays were performed in triplicates and values are shown as mean ± S.D. of proliferation percentage against PHA 40 µg/ml. * indicates significantly greater (p<0.05) than that of negative control and DNA/no EP groups.

The results of this experiment indicate that compared to DNA injection alone, with respect to cellular immune responses, in vivo EP using the single-needle device of the current invention increased lymphocyte proliferation by 2 to 3 fold. This is comparable over known current immunization technology, potentially providing substantial advantages for eliciting immunity over presently used methods and devices.

### Experiment VII

In this experiment, yet another antigen was tested for elicitation of antibody production in a test animal to show that the single-needle electrode is capable of electroporating cells sufficient to bring about appropriate immune responses. Here, human IgG3 antigen construct, pLNOH2hg3-hk-IEd (Aldevron, ND), expressing human IgG3, were used for plasmid immunization so as to test for antibody reaction against the human IgG3. In this experiment, the quadriceps muscle of 3-4 kg NZW male rabbits were primed at day 0 and boosted at day 14. The IgG3 plasmid DNA, dissolved in saline, were injected at days 0 and 14 each followed by pulsing using the same single-needle electrode as described in example VI. Amounts of plasmid DNA used were 100ug of pLNOH2hg3-hk-IED in 200 ul saline.

Serum samples were collected at 2, 3, 4, 5 weeks after the first immunization by ear artery bleeds. The expression of rabbit antibody titer (rabbit IgG-anti human IgG3) in sera was analyzed by enzyme-linked immunoassays (ELISA) conducted as well described in the immunologic arts.

Results showed an enhanced rabbit IgG-anti human IgG3 serum titers following DNA vaccination with electroporation in rabbit quadriceps. Specifically, rabbits were primed and boosted in 2 weeks with pLNOH2hg3-hk-IED in the absence or presence of electroporation with 125mA, 250 mA or high & low voltages (HL, 50V/200 microseconds & 10V/ 200 milliseconds). In Figure **29****,** data from individual rabbits were pooled from two identical measurements with median value indicated by a horizontal bar. The time course of rabbit IgG-anti human IgG3 serum titers are shown for groups receiving no electroporation, electroporation at 125mA, electroporation at 250mA, and group using the high and low voltage pulses. The antibody titers are significantly higher than that of DNA injection without electroporation (*indicates p<0.05).

### Experiment VIII

In this set of experiments expression pattern of the transgene was examined in rabbit muscle following Electroporation enhanced transfection at different current levels using the same single needle electrode as described in example VI and VII.

Plasmid constructs for the experiment were gWiz-GFP, expressing Green Fluorescent protein under the human control of the CMV promoter/enhancer (obtained from Aldevron LLC, Fargo, ND).

In NZW male rabbits (3.0-4.0 Kg), quadriceps muscles were injected with the DNA and electroporated. In each treatment, 200ug of g Wiz-GFP in 200 ul saline was injected per site of treatment in each rabbit followed by electroporation through the same single-needle electrode. The electrical pulse in each instance of prime and boost comprised 2 pulses at constant current of 75, 125 or 250mA corresponding to 8V, 15V or 25V, 60 ms pulse length, 250 ms interval between the pulses that gives about 4 Hz frequency.

Rabbits were euthanized and sacrificed in day 3, and muscles were taken and sectioned at 1.5mm thickness for image analyses using Olympus OV-100 fluorescence microscope (objective 0.14x),

Results of this experiment show that GFP expression in rabbit quadriceps was dramatically improved with DNA injection followed by electroporation using the single-needle electrode. As shown in Figures **30A** and **30B** (combined fluorescence and light field and fluorescence only, respectively), muscle from one quadriceps injected with DNA alone without electroporation followed by GFP expression gave no detectable expression of DNA.

However, when DNA injection was followed by electroporation using any of two 60 ms pulses at 8 V, 12.5 V, or 25V, the expression of GFP was clearly visible through four continuous slices of about 1.5 mm thickness corresponding to a barrel or generally cylindrical shaped transfected area of 4-8 mm diameter x 20 mm length corresponding to the length of the needle track. Shown are representative examples at Electroporation at 8V (Figures **30C** (combined light/fluoescence) and **30D** (fluorescence only), electroporation at 15V (Figures **30E** (combined light/fluoescence) and 30F (fluorescence only)) and electroporation at 25V (Figures **30G** (combined light/fluoescence) and **30H** (fluorescence only)).

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the compositions and methods and in the steps or in the sequence of steps of the method described herein without departing from the scope of the invention. More specifically, the described embodiments are to be considered in all respects only as illustrative and not restrictive. All similar substitutes and modifications apparent to those skilled in the art are deemed to be within the scope of the invention as defined by the appended claims.

All patents, patent applications, and publications mentioned in the specification are indicative of the levels of those of ordinary skill in the art to which the invention pertains.

The invention illustratively described herein suitably may be practiced in the absence of any element(s) not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and there is no intention that use of such terms and expressions imply excluding any equivalents of the features shown and described in whole or in part thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A single-needle electrode (10) for reversible electroporation of tissue *in vivo*, comprising:
a. an elongate hollow delivery tube capable of penetrating a body tissue, wherein the delivery tube comprises at least one each of an anode and a cathode (21 a, 21b, 31a, 31b, 41, 42, 51 a, 51b, 90, 101) disposed on at least a portion of an outer surface of said delivery tube; and
b. electrically conductable conduits capable of connecting each of said anode and cathode (2 1 a, 21b, 31a, 31b, 41, 42, 51 a, 51b, 90, 101) to an electrical energy source;
**characterised in that** the anode and the cathode are elongate and are spaced and electrically isolated from one another and situated parallel with respect to one another, and further **characterised in that** the delivery tube is a fenestrated needle having delivery ports along the length of the needle, wherein when said delivery tube is inserted into tissue and when said anode(s) and said cathode(s) (21a, 21b, 31a, 31b, 41, 42, 51a, 51b, 90, 101) are energized by said energy source, an electric field is generated to reversibly electroporate cells in a treatment zone surrounding said delivery tube so as to allow said cells to take up an active ingredient in a fluid composition delivered through said delivery tube.

2. The device according to claim 1 wherein said delivery tube comprises a hypodermic needle sized to the gauge of an injection needle selected from the group consisting of 20 gauge, 21 gauge, 22 gauge, 23 gauge, 24 gauge, 25 gauge, 26 gauge, 27 gauge, 28 gauge and 29 gauge.

3. The device according to claim 2 wherein said delivery tube is between 3mm and 1.5 cm in length.

4. The device according to claim 2 wherein said delivery tube is between 1.0 and 3 cm in length.

5. The device according to claim 1 wherein said delivery tube is electrically insulated from each anode and cathode (21 a, 21 b, 31 a, 31b, 41, 42, 51 a, 51b, 90, 101).

6. The device according to claim 5 wherein said anode and said cathode (31a, 31b, 41, 42) spiral around said tube while maintaining a parallel relation to one another in a spiral plane about said delivery tube.

7. The device according to claim 1 further comprising an expandable or contractible reservoir.

8. The device according to claim 7 wherein said reservoir has a variable volume capacity selected from the group consisting of 0.0 to 0.5ml, 0.0 to 1ml, 0.0 to 3ml, and 0.0 to 5ml.

9. The device according to claim 1 further comprising an electrical energy source wherein said electrical energy source is an electroporation pulse generator.

10. The device according to claim 1 wherein said tissue comprises a body tissue type or organ selected from the group consisting of skin, subcutaneous tissue, intradermal tissue, subdermal tissue, skeletal muscle, striated muscle, smooth muscle, organs, heart, breast, lung, pancreas, liver, spleen, and mucosa.

11. The device according to claim 9 wherein said electrical source of energy is supplied by a generator that is pulsed such that the nominal voltage per pulse is between 1 to 200 V.

12. The device according to claim 9 wherein said generator is pulsed at a constant current in the range of from 1 to 1000 mAmps.

13. The device according to claim 12 wherein said constant current range is selected from the group consisting of between 10 and 40, 25 and 100, 50 and 150, 125 and 200, 175 and 250, 225 and 300, 250 and 300, 300 and 400, 400-600, and 600 to 1000 mAmps.

14. The device according to claim 9 wherein said generator is pulsed at a frequency selected from between the range 1 to 10,000 Hz.

15. The device according to claim 9 wherein said generator is pulsed for a time length between about 0.1 µs to 1000 ms.

## Patentansprüche

1. Einzelnadel-Elektrode (10) für die reversible Elektroporation von Gewebe *in vivo,* umfassend:
a. ein längliches hohles Zuführröhrchen, das zum Eindringen in ein Körpergewebe fähig ist, wobei das Zuführröhrchen zumindest eines von jeweils einer Anode und einer Kathode (21a, 21b, 31a, 31b, 41, 42, 51a, 51b, 90, 101) umfasst, die an zumindest einem Teil einer äußeren Oberfläche des Zuführröhrchens angebracht sind; und
b. elektrisch leitfähige Leitungen, die zum Anschließen jeweils der Anode und der Kathode (21a, 21b, 31a, 31b, 41, 42, 51a, 51b, 90, 101) an eine elektrische Energiequelle befähigt sind;
**dadurch gekennzeichnet, dass** die Anode und die Kathode länglich sind und voneinander beabstandet und elektrisch isoliert sind und in Bezug aufeinander parallel angeordnet sind, und außerdem **dadurch gekennzeichnet, dass** das Zuführröhrchen eine gefensterte Nadel mit Zuführanschlüssen entlang der Länge der Nadel ist, wobei dann, wenn das Zuführröhrchen in Gewebe eingeführt ist und wenn die Anode(n) und die Kathode(n) (21a, 21b, 31a, 31b, 41, 42, 51 a, 51 b, 90, 101) durch die Energiequelle unter Spannung gesetzt werden, ein elektrisches Feld erzeugt wird, um Zellen in einer Behandlungszone rings um das Zuführröhrchen reversibel zu elektroporieren, um jenen Zellen das Aufnehmen eines Wirkstoffs in einer durch das Zuführröhrchen überbrachten flüssigen Zusammensetzung zu ermöglichen.

2. Vorrichtung gemäß Anspruch 1, wobei das Zuführröhrchen eine hypodermale Nadel umfasst, die entsprechend der Gauge-Dicke einer Injektionsnadel, ausgewählt aus der Gruppe, bestehend aus 20 Gauge, 21 Gauge, 22 Gauge, 23 Gauge, 24 Gauge, 25 Gauge, 26 Gauge, 27 Gauge, 28 Gauge und 29 Gauge, größenbemessen ist.

3. Vorrichtung gemäß Anspruch 2, wobei das Zuführröhrchen zwischen 3 mm und 1,5 cm lang ist.

4. Vorrichtung gemäß Anspruch 2, wobei das Zuführröhrchen zwischen 1,0 mm und 3 cm lang ist.

5. Vorrichtung gemäß Anspruch 1, wobei das Zuführröhrchen vor jeder Anode und Kathode (21 a, 21 b, 31 a, 31 b, 41, 42, 51 a, 51b, 90, 101) elektrisch isoliert ist.

6. Vorrichtung gemäß Anspruch 5, wobei sich die Anode und die Kathode (31a, 31b, 41, 42) spiralförmig um das Röhrchen winden, unter Beibehaltung einer parallelen Beziehung zueinander in einer Ebene der Spirale um dieses Zuführröhrchen.

7. Vorrichtung gemäß Anspruch 1, außerdem umfassend einen ausdehnbaren oder zusammenziehbaren Speicher.

8. Vorrichtung gemäß Anspruch 7, worin der Speicher eine variable Volumenkapazität, ausgewählt aus der Gruppe, bestehend aus 0,0 bis 0,5 ml, 0,0 bis 1 ml, 0,0 bis 3 ml und 0,0 bis 5 ml, aufweist.

9. Vorrichtung gemäß Anspruch 1, weiters umfassend eine elektrische Energiequelle, wobei diese elektrische Energiequelle ein Elektroporations-Pulsgenerator ist.

10. Vorrichtung gemäß Anspruch 1, wobei das Gewebe einen Körpergewebstyp oder ein Organ umfasst, ausgewählt aus der Gruppe bestehend aus Haut, subkutanem Gewebe, intradermalem Gewebe, subdermalem Gewebe, Skelettmuskel, quergestreiftem Muskel, glattem Muskel, Organen, Herz, Brust, Lunge, Bauchspeicheldrüse, Leber, Milz und Schleimhaut.

11. Vorrichtung gemäß Anspruch 9, wobei die elektrische Quelle der Energie mittels eines Generators bereitgestellt wird, der so gepulst wird, dass die nominale Spannung pro Puls zwischen 1 und 200 V beträgt.

12. Vorrichtung gemäß Anspruch 9, wobei der Generator bei einem Konstantstrom im Bereich von 1 bis 1000 mA gepulst wird.

13. Vorrichtung gemäß Anspruch 12, wobei dieser Konstantstrombereich ausgewählt ist aus der Gruppe, bestehend aus zwischen 10 und 40, 25 und 100, 50 und 150, 125 und 200, 175 und 250, 225 und 300, 250 und 300, 300 und 400, 400 bis 600 und 600 bis 1000 mA.

14. Vorrichtung gemäß Anspruch 9, wobei der Generator bei einer Frequenz, ausgewählt aus dem Bereich von 1 bis 10.000 Hz gepulst wird.

15. Vorrichtung gemäß Anspruch 9, wobei der Generator für eine Zeitperiode von zwischen etwa 0,1 µs und 1000 ms gepulst wird.

## Revendications

1. Électrode à aiguille unique (10) pour l'électroporation réversible d'un tissu *in vivo*, comprenant :
a. un tube de distribution, creux et allongé, capable de pénétrer dans un tissu corporel, dans lequel le tube de distribution comprend au moins une anode et au moins une cathode (21a, 21b, 31a, 31b, 41, 42, 51a, 51b, 90, 101) disposées sur au moins une partie d'une surface externe dudit tube de distribution ; et
b. des conduits pouvant être électro-conducteurs capables de connecter ladite anode et ladite cathode (21a, 21b, 31a, 31b, 41, 42, 51a, 51b, 90, 101) à une source d'énergie électrique ;
**caractérisée en ce que** l'anode et la cathode sont allongées et sont espacées et isolées électriquement l'une de l'autre et parallèles l'une par rapport à l'autre, et **caractérisée en outre en ce que** le tube de distribution est une aiguille fenêtrée ayant des orifices de distribution le long de la longueur de l'aiguille, et quand ledit tube de distribution est inséré dans un tissu et quand ladite (lesdites) anode(s) et cathode(s) (21a, 21b, 31a, 31b, 41, 42, 51a, 51b, 90, 101) sont alimentées par ladite source d'énergie, un champ électrique est généré pour permettre l'électroporation réversible des cellules dans une zone de traitement entourant ledit tube de distribution afin de permettre auxdites cellules d'absorber un principe actif dans une composition fluide délivrée par l'intermédiaire dudit tube de distribution.

2. Dispositif selon la revendication 1, dans lequel ledit tube de distribution comprend une aiguille hypodermique ayant le calibre d'une aiguille d'injection choisi dans le groupe constitué des calibres 20, 21, 22, 23, 24, 25, 26, 27, 28 et 29.

3. Dispositif selon la revendication 2, dans lequel la longueur dudit tube de distribution est de 3 mm à 1,5 cm.

4. Dispositif selon la revendication 2, dans lequel la longueur dudit tube de distribution est de 1,0 cm à 3 cm.

5. Dispositif selon la revendication 1, dans lequel ledit tube de distribution est électriquement isolé de l'anode et de la cathode (21a, 21b, 31a, 31b, 41, 42, 51a, 51b, 90, 101).

6. Dispositif selon la revendication 5, dans lequel ladite anode et ladite cathode (31a, 31b, 41, 42) sont enroulés en spirale autour dudit tube tout en conservant une relation parallèle l'une par rapport à l'autre dans un plan en spirale autour dudit tube de distribution.

7. Dispositif selon la revendication 1, comprenant en outre un réservoir dilatable ou contractible.

8. Dispositif selon la revendication 7, dans lequel ledit réservoir possède une capacité de volume variable choisi dans le groupe constitué de 0,0 ml à 0,5 ml, de 0,0 ml à 1 ml, de 0,0 ml à 3 ml et de 0,0 ml à 5 ml.

9. Dispositif selon la revendication 1, comprenant en outre une source d'énergie électrique, dans laquelle ladite source d'énergie électrique est un générateur d'impulsion d'électroporation.

10. Dispositif selon la revendication 1, dans lequel ledit tissu comprend un type de tissu corporel ou un organe choisi dans le groupe constitué de la peau, d'un tissu sous-cutané, d'un tissu intradermique, d'un tissu sous-dermique, d'un muscle squelettique, d'un muscle strié, d'un muscle lisse, des organes, du coeur, du sein, du poumon, du pancréas, du foie, de la rate et des muqueuses.

11. Dispositif selon la revendication 9, dans lequel ladite source d'énergie électrique est fournie par un générateur qui est pulsé de façon que la tension nominale par impulsion soit située entre 1 V et 200 V.

12. Dispositif selon la revendication 9, dans lequel ledit générateur est pulsé à un courant constant situé dans la plage allant de 1 mA à 1 000 mA.

13. Dispositif selon la revendication 12, dans lequel ladite plage de courants constants est choisie dans le groupe constitué de 10 à 40, 25 à 100, 50 à 150, 125 à 200, 175 à 250, 225 à 300, 250 à 300, 300 à 400, 400 à 600 et 600 à 1 000 mA.

14. Dispositif selon la revendication 9, dans lequel ledit générateur est pulsé à une fréquence choisie dans la plage de 1 Hz à 10 000 Hz.

15. Dispositif selon la revendication 9, dans lequel ledit générateur est pulsé pendant une durée d'environ 0,1 µs à 1 000 ms.
